# EUROPEAN PATENT APPLICATION

(11) **EP 4 442 279 A1**
(43) Date of publication of application: **09.10.2024**
(21) Application number: 22901178.8
(22) Date of filing: 24.11.2022
(51) Int. Cl.: A61K 45/00, A61P 1/00, A61P 37/04, A23L 33/135, A61K 35/74

(54) **IMMUNOSTIMULATORY COMPOSITION**

(30) Priority: 02.12.2021 JP 2021196561
(71) Applicant: Shinkura, Reiko, Tokyo 113-8654 (JP)
(72) Inventor: GAO Peng, Tokyo 113-8654 (JP); SHINKURA Reiko, Tokyo 113-8654 (JP)
(74) Representative: Grünecker Patent- und Rechtsanwälte PartG mbB
(86) International application number: PCT/JP2022/043424
(87) International publication number: WO 2023/100745

(57) **Abstract**

The present disclosure relates to a composition for inducing migration of activated B cells to a germinal center, the composition including a substance that induces CD11b expression in B cells, a production method therefor, or use thereof. In one aspect, the present disclosure relates to immunostimulation and maintenance or improvement of mucosal flora using the substance that induces the CD11b expression in B cells.

## Description

### Technical Field

The present disclosure relates to a composition for inducing migration of activated B cells to a germinal center, the composition including a substance that induces CD11b expression in B cells, a production method therefor, or use thereof. In one aspect, the present disclosure relates to immunostimulation and maintenance or improvement of mucosal flora using the substance that induces the CD11b expression in B cells.

### Background Art

Research on the behavior of B cells and germinal centers (GCs) during immune responses is motivated by their potential to aid in vaccine development (Non Patent Literatures 1 and 2). One of the important criteria in evaluating efficacy of a vaccine is whether the vaccine can efficiently induce germinal center (GC) responses and generate high-affinity, long-lived plasma cells (PCs) and memory B cells, thus protecting the host from invading pathogens (Non Patent Literatures 1 and 2). During a GC response, B cells undergo 3 stages: pre-GC, GC and post-GC

(Non Patent Literatures 3 to 5). Upon antigenic stimulation, newly activated naive B cells and activated memory B cells directly differentiate into extrafollicular short-lived PCs or migrate to the interfollicular (IF) region and establish stable interactions with activated T cells (Non Patent Literatures 3 to 8). After interaction between T-B cells, some B cells differentiate into low-affinity PCs, and other B cells (pre-GC B cells) migrate to GCs (Non Patent Literatures 3 and 4). These pre-GC B cells first migrate to the dark zone (DZ) of GC and develop into GC B cells. B cells rapidly proliferate, undergo somatic hypermutation (SHM) to change the affinity of the B cell receptor (BCRs), and bind to its specific antigen (Non

Patent Literatures 3 and 4). B cells with mutations in the immunoglobulin (Ig) genes then migrate from the DZ to the light zone (LZ), where affinity selection takes place with the help of T follicular helper (T_{FH}) cells (Non Patent Literatures 3, 4, and 8). B cells with a BCR with high affinity for the antigen are selected as post-GC B cells, exit the GC, and eventually differentiate into long-lived PCs and memory B cells, whereas unselected B cells can return to the DZ of the GC and further accumulate SHM for BCR with higher affinity (Non Patent Literature 7).

It has been reported that efficient activation of dendritic cells (DCs) is essential for causing the GC response (Non Patent Literature 3). Therefore, studies so far have focused on selecting the optimal adjuvant for stimulating DCs. However, Lycke et al. revealed that in mice depleted of DCs, B cells collect antigens from microfold (M) cells and further migrate to GC (Non Patent Literature 9), indicating that DC activation is not necessary for the invasion into GCs. Even in a case where DCs are properly primed, only a small population of activated B cells can develop into pre-GC B cells and enter GCs (Non Patent Literatures 4 and 6). Recent studies have reported that irf4, a transcription factor essential for GC formation, is highly expressed in pre-GC B cells (Non patent Literatures 10 and 11). However, specific surface markers for pre-GC B cells are unknown. Therefore, the criteria for pre-GC B cells and what types of activated B cells enter the GC and produce BCRs with high affinity for specific antigens have not been well understood.

### Citation List

### Non Patent Literature

Non Patent Literature 1: Turner, J. S. et al. Human germinal centres engage memory and naive B cells after influenza vaccination. Nature. 586, 127-132 (2020).
Non Patent Literature 2: Andersen, T. K. et al. Enhanced germinal center reaction by targeting vaccine antigen to major histocompatibility complex class II molecules. npj Vaccines. 4, 9 (2019).
Non Patent Literature 3: Mesin, L., Ersching, J., & Victora, G. D. Germinal center B cell dynamics. Immunity. 45.3, 471-482 (2016).
Non Patent Literature 4: Kurosaki, T., Kometani, K., & Ise, W. Memory B cells. Nature Reviews Immunology. 15.3, 149-159 (2015) .
Non Patent Literature 5: Chen, K., Magri, G., Grasset, E. K. & Cerutti, A. Rethinking mucosal antibody responses: IgM, IgG and IgD join IgA. Nature Reviews Immunology. 20, 427-441 (2020).
Non Patent Literature 6: Mesin, L. et al. Restricted clonality and limited germinal center reentry characterize memory B cell reactivation by boosting. Cell. 180.1, 92-106 (2020) .
Non Patent Literature 7: Chen, H. et al. BCR selection and affinity maturation in Peyer's patch germinal centres. Nature. 582, 421-425 (2020).
Non Patent Literature 8: Ise, W. et al. T follicular helper cell-germinal center B cell interaction strength regulates entry into plasma cell or recycling germinal center cell fate. Immunity. 48.4, 702-715 (2018).
Non Patent Literature 9: Komban, R.J. et al. Activated Peyer's patch B cells sample antigen directly from M cells in the subepithelial dome. Nat Commun. 10, 2423 (2019). Non Patent Literature 10: Song, S. & Matthias, P. D. The Transcriptional Regulation of Germinal Center Formation. Front Immunol. 10.3389 (2018).
Non Patent Literature 11: Willis, SN. et al. Transcription factor IRF4 regulates germinal center cell formation through a B cell-intrinsic mechanism. J Immunol. 192(7), 3200-6 (2014).

### Summary of Invention

Activated B cells can enter germinal centers (GCs) to undergo affinity maturation and produce high-affinity antibodies. However, it remained unclear what kind of activated B cells enter GCs. Here, the inventors discovered a small population of CD11b⁺IgA⁺ B cells located outside GCs. When these CD11b⁺IgA⁺ B cells were injected into mice, the cells entered GCs after 40 hours. From this result, it was suggested that CD11b is a new surface marker for pre-GC IgA⁺ B cells. Furthermore, independent of dendritic cell activation, CD11b expression on B cells was found to be induced by bacterial antigens such as pam3CSK4 and heat-killed E. coli in vitro. Moreover, when pam3CSK4 or heat-killed E. coli was orally administered, the number of GC B cells increased within 2 days, and the mucosal antigen-specific IgA response was enhanced. The results presented in the present disclosure demonstrate that the induction of CD11b in B cells is a promising marker for selecting effective mucosal vaccine adjuvants.

Based on the above findings, the inventors of the present disclosure have found that a substance that induces CD11b expression in B cells can act as an effective adjuvant for mucosal vaccines and induce the production of high-affinity mucosal IgA antibodies, which is an action that can stimulate immunity in mucosa or the like and induce immunity against various antigens derived from outside the body, and this effect leads to maintenance of appropriate mucosal flora or improvement of mucosal flora, thus completing the present disclosure.

In one aspect, the present disclosure provides a composition for inducing migration of activated B cells to a germinal center, the composition including a substance that induces CD11b expression in B cells.

In one aspect, the present disclosure provides a method for inducing migration of activated B cells to a germinal center, the method including preparing a composition comprising a substance that induces CD11b expression in B cells and bringing the substance into contact with B cells, in which the CD11b expression in the B cells is induced, and the contacts between the B cells and T cells and the B cells and dendritic cells are induced.

More specifically, the present disclosure provides the following.

### [Item 1]

A composition for inducing migration of activated B cells to a germinal center, the composition including a substance that induces CD11b expression in B cells.

### [Item 2]

The composition according to item 1, in which the substance that induces the CD11b expression in B cells is a bacterium.

### [Item 3]

The composition according to item 2, in which the substance that induces the CD11b expression in B cells is a bacterium of the class γ Proteobacteria or class Bacteroidia.

### [Item 4]

The composition according to item 3, in which the substance that induces the CD11b expression in B cells is Escherichia coli, Salmonella enterica, Klebsiella pneumoniae, Bacteroides vulgatus, or a combination thereof.

### [Item 5]

The composition according to any one of items 1 to 4, which is for treatment of a subject with a disease, disorder, or condition that is treated by activation of the migration of activated B cells to a germinal center.

### [Item 6]

The composition according to any one of items 1 to 4, which is used for immunostimulation, maintenance or improvement of health, maintenance or improvement of mucosal flora, or treatment of an infectious disease.

### [Item 7]

The composition according to any one of items 1 to 4, which is a vaccine adjuvant.

### [Item 8]

The composition according to any one of items 1 to 7, which is administered mucosally.

### [Item 9]

The composition according to any one of items 1 to 6, which is in a form selected from the group consisting of a medicine, a food, a feed, a food additive, a feed additive, and a raw material composition thereof.

### [Item 10]

The composition according to item 1, which is for administration with an antigen.

### [Item 11]

The composition according to item 10, in which the composition is administered simultaneously with the antigen.

### [Item 12]

The composition according to item 10, in which the composition is administered separately from administration of the antigen.

### [Item 13]

The composition according to item 1, which is a vaccine composition including an antigen.

### [Item 14]

The composition according to items 10 to 13, in which the antigen is a viral antigen, a bacterial antigen, a parasitic antigen, or a combination thereof.

### [Item 15]

A method for inducing migration of activated B cells to a germinal center, the method including preparing a composition including a substance that induces CD11b expression in B cells and bringing the substance into contact with B cells, in which the CD11b expression is induced in the B cells, and the contacts between the B cells and T cells and the B cells and dendritic cells are induced.

Furthermore, in another aspect, the present disclosure provides the following.

### [Item 16]

A method for treating a subject with a disease, disorder, or condition that is treated by activation of migration of activated B cells to a germinal center, the method including administering a composition including a substance that induces CD11b expression in B cells to the subject.

### [Item 17]

The method according to item 16, in which the substance that induces the CD11b expression in B cells is a bacterium.

### [Item 18]

The method according to item 16, in which the substance that induces the CD11b expression in B cells is a bacterium of the class γ Proteobacteria or class Bacteroidia.

### [Item 19]

The method according to item 16, in which the substance that induces the CD11b expression in B cells is Escherichia coli, Salmonella enterica, Klebsiella pneumoniae, Bacteroides vulgatus, or a combination thereof.

### [Item 20]

The method according to item 16, in which the treatment of a disease, disorder, or condition that is treated by the activation of the migration of activated B cells to a germinal center is immunostimulation, maintenance or improvement of health, maintenance or improvement of mucosal flora, or treatment of an infectious disease.

### [Item 21]

The method according to item 16, in which the composition is formulated as a vaccine adjuvant.

### [Item 22]

The method according to item 16, in which the composition is administered mucosally.

### [Item 23]

The method according to item 16, in which the composition is in a form selected from the group consisting of a medicine, a food, a feed, a food additive, a feed additive, and a raw material composition thereof.

### [Item 24]

The method according to item 16, in which the composition is administered together with an antigen.

### [Item 25]

The method according to item 16, in which the composition is administered simultaneously with an antigen.

### [Item 26]

The method according to item 16, in which the composition is administered separately from the administration of an antigen.

### [Item 27]

The method according to item 16, in which the composition is a vaccine composition including an antigen.

### [Item 28]

The method according to items 24 to 27, in which the antigen is a viral, bacterial, fungal, or parasitic antigen.

### Brief Description of Drawings

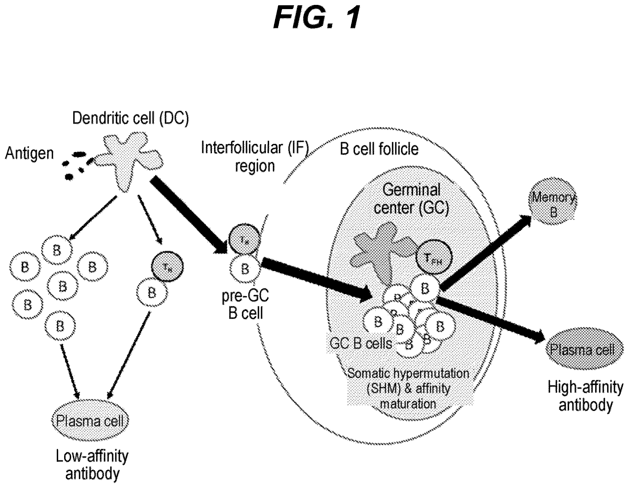

Fig. 1 is a schematic diagram showing that only a portion of B cells invade a germinal center.

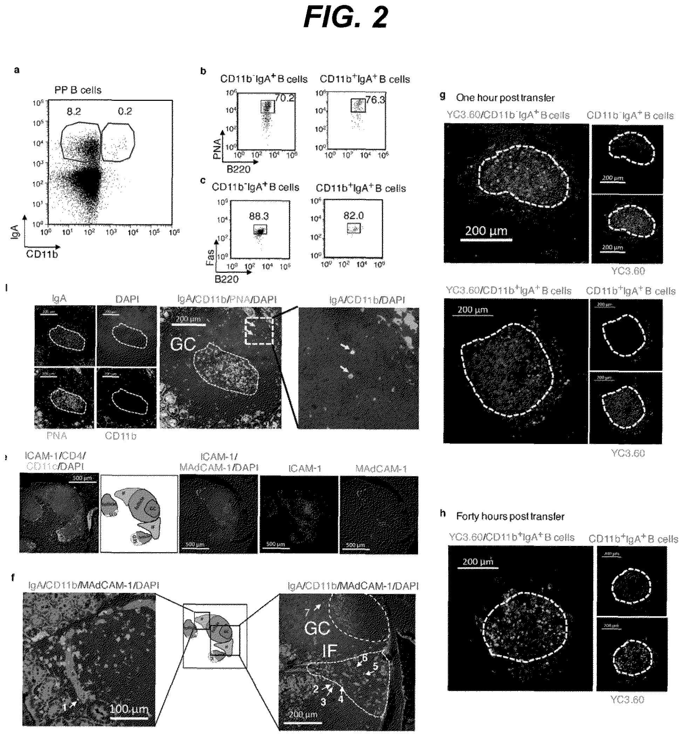

Fig. 2 is a diagram showing that CD11b⁺IgA⁺ PP B cells are located outside a GC and are moving toward the GC. a, Representative flow cytometry data showing expression of IgA and CD11b in PP B220⁺ B cells in WT mice. b and c, Flow cytometry of GC B cells in CD11b⁺IgA⁺ PP B cells stained with B220 and PNA (b) or B220 and Fas (c). a to c, Numbers indicate the percentage of cells. d, Micrographs of PP cross sections stained with PNA, CD11b, IgA, and DAPI. Two CD11b⁺IgA⁺ double positive cells (yellow arrows) were observed outside the GC (dashed circle). e, Images of SED, IF, and GC regions stained with ICAM-1, CD11c, CD4 (left photo), MAdCAM-1 (right photo), and DAPI. Each region is marked in the schema in the center. Scale bar, 500 um. f, The images show that CD11b⁺IgA⁺ B cells (arrows, marked 1 to 7) are present in the indicated regions. The GC and IF regions are indicated by dashed lines. g, CD11b⁻IgA⁺ B cells and CD11b⁺IgA⁺ B cells were directly injected into PPs of IgA-Cre/YC3.60^{flox} mice, respectively. Representative images showing the locations of CD11b⁺IgA⁺ PP B cells (top) or CD11b⁻IgA⁺ PP B cells (bottom) transferred into the PPs of the IgA-Cre/YC3.60^{flox} mice 1 hour after the injection. The GC boundaries (dashed lines) are the regions enriched with IgA-YC3.60. Scale bar, 200 µm. h, 40 hours after the injection, the PP with the injected CD11b⁺IgA⁺ B cells was observed under a microscope. Scale bar, 200 um. Data are representative of 3 independent experiments.

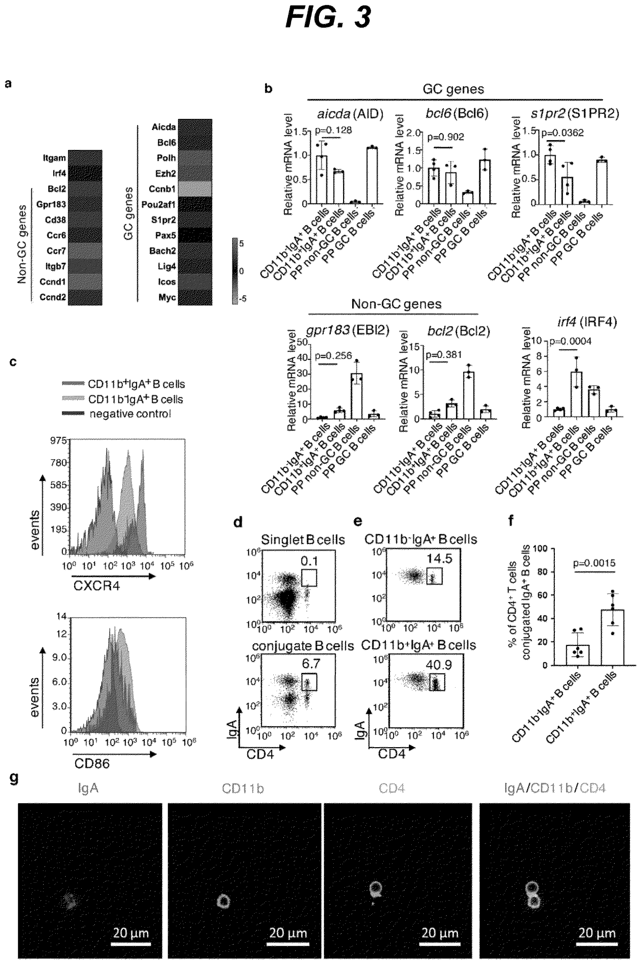

Fig. 3 is a diagram showing that CD11b⁺IgA⁺ B cells are pre-GC B cells that interact with CD4⁺ T cells. a, Heat maps showing logarithmic fold changes in the expression levels of the indicated genes (CD11b⁺IgA⁺ B cells/CD11b⁻IgA⁺ B cells) obtained by microarray analysis. b, The expression levels of the indicated GC-specific genes, non-GC-specific genes, and pre-GC genes were analyzed by qPCR and normalized to the expression level of the β-actin gene. The average relative expression level in the CD11b⁻IgA⁺ B cells was set to be 1. Each spot represents the average of three technical replicates. The bar graphs represent the mean (± SD) of 3 or 4 independent measurements. c, Flow cytometry histograms showing the expression of the CXCR4 and CD86 surface markers on the CD11b⁺IgA⁺ B cells and CD11b⁻IgA⁺ B cells. d, Flow cytometry data of singlet IgA⁺ B cells and conjugated CD4⁺ IgA⁺ cells. e, Flow cytometry data of CD4⁺ T cells conjugated with CD11b⁻IgA⁺ and CD11b⁺IgA⁺ PP B cells. f, Percentages of CD4⁺ T cells conjugated with CD11b⁺IgA⁺ and CD11b⁻IgA⁺ PP B cells. The bar graphs represent the mean (± SD) of 6 independent measurements. g, Photographs of the appearances of the CD11b⁺IgA⁺ PP B cells conjugated with the CD4⁺ T cells. Scale bar, 20 µm.

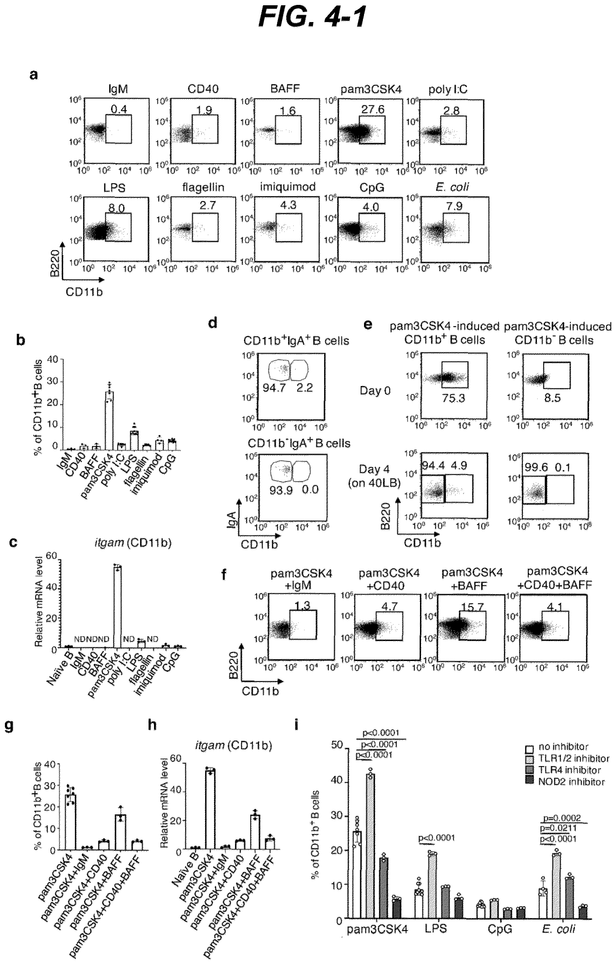

Fig. 4-1 is a diagram showing that bacterial antigens induce CD11b expression in B cells. a and f, Data obtained by flow cytometric analysis of the CD11b expression in splenic naive B cells subjected to the indicated stimulation for 3 days. b and g, Percentage of CD11b⁺ B cells for each stimulation, c and h, Data obtained by qPCR analysis of itgam in B cells isolated after applying the indicated stimulation, d, Flow cytometry data of CD11b⁺IgA⁺ and CD11b⁻IgA⁺ PP B cells isolated after being cultured on 40LB cells for 1 day. e, The CD11b expression induced by Pam3CSK4 was lost by the culture on the 40LB cells. i, Percentages of CD11b⁺ B cells for each stimulation with the indicated inhibitors. b, c, g, h, and i, The bar graphs represent the mean (± SD) of at least 3 independent measurements. b, c, g, and h, Data were analyzed by one-way ANOVA and Tukey's multiple comparison.

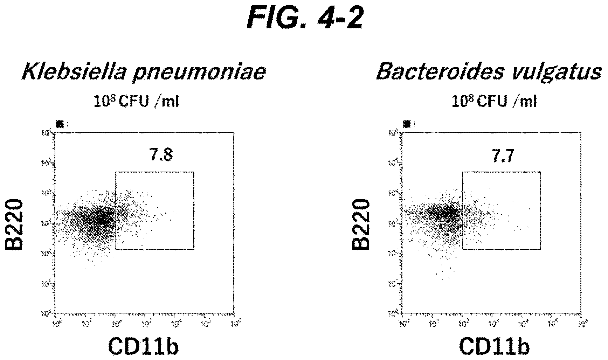

Fig. 4-2 is a diagram showing that heat-sterilized Klebsiella pneumoniae, bacteria of the class Proteobacteria, and Bacteroides vulgatus, bacteria of the class Bacteroidia, induce the CD11b expression in B cells.

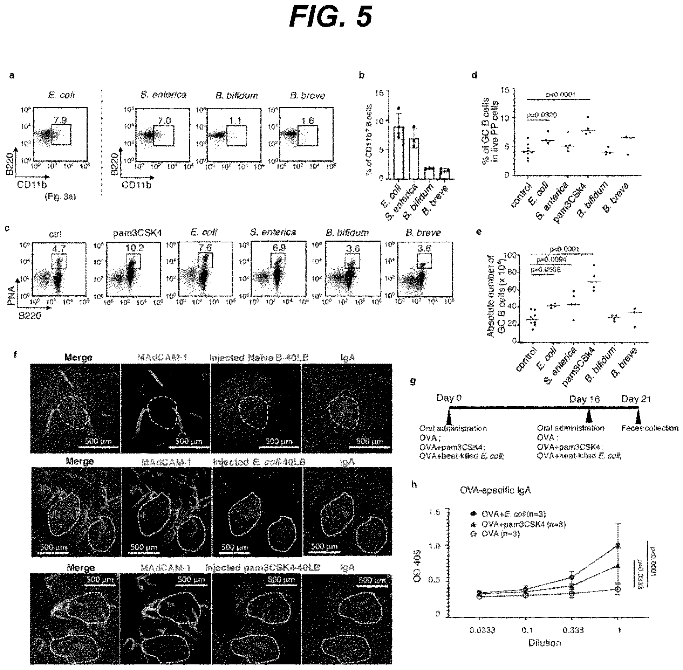

Fig. 5 is a diagram showing that CD11b-inducing bacterial antigens enhance the pre-existing GC response in PP. a, Flow cytometry data of the CD11b expression in splenic naive B cells stimulated by the indicated stimuli for 3 days. b Percentage of CD11b⁺ B cells for each stimulus. c to e, Balb/c mice were orally administered with the indicated heat-killed bacteria, pam3CSK4, or PBS (control). 2 days later, flow cytometry data of PNA^{hi}B220⁺ GC B cells (c) and the percentage (d) and the absolute number (e) of PNA^{high}B220⁺ PP GC B cells were calculated for each mouse, f, The indicated iGB cells were isolated, labeled, and then independently injected intravenously into mice. Scale bar, 500 µm. g, Immunization schedule. h, OVA-specific IgA after the immunization. b, d, e, and h, The bar graphs represent the mean (± SD) of at least 3 independent measurements. d and e, Data were analyzed by one-way ANOVA and Tukey's multiple comparison. h, Data were analyzed by two-way ANOVA and Tukey's multiple comparison.

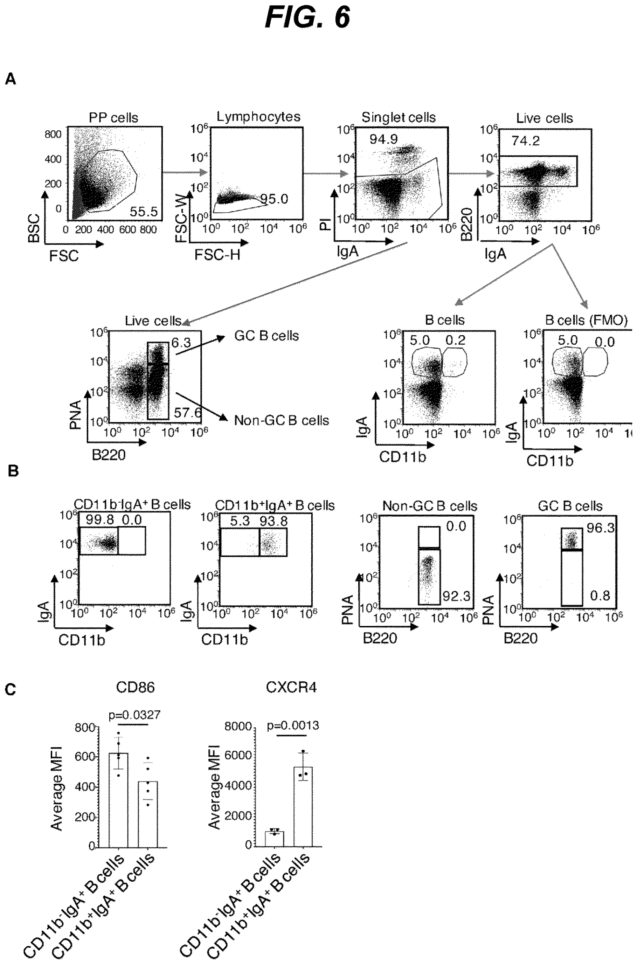

Fig. 6 is a diagram showing the presence of CD11b⁺IgA⁺ B cells in mouse PP. a, Flow cytometry analysis was performed using 8 to 12-week-old WT Balb/c mice. Doublet cells were discriminated using an FSC-H/FSC-W gate. Dead cells were excluded by propidium iodide (PI) staining. The expression of IgA and CD11b in B220+ B cells was analyzed. In order to confirm the expression of CD11b, a fluorescence minus one (FMO) sample was prepared as a negative control. In live cells, GC B cells represent PNA^{high}B220⁺ cells, and non-GC B cells represent PNA^{low}B220⁺ cells. Representative data were obtained from at least 20 experiments. b, The purity of the sorting was confirmed to be at least 90%. c, Average mean fluorescence intensity (MFI) analysis of CD86 expression and CXCR4 expression in CD11b⁺IgA⁺ PP B cells and CD11b⁻IgA⁺ PP B cells. The bar graphs represent the mean (± SD) of 5 independent measurements performed using WT mice. Data were compared using a two-tailed Student's t test.

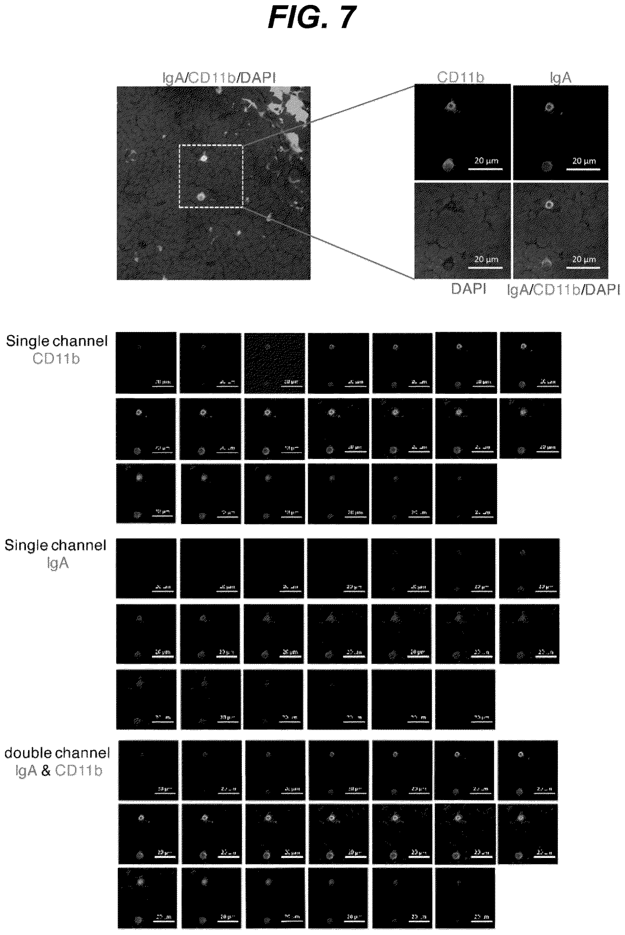

Fig. 7 is a diagram showing immunohistochemical confocal images for confirming the expression of CD11b and IgA. A selected PP cross section was stained with DAPI, CD11b, and IgA. The indicated portion (dashed square) was amplified, and the overlap between CD11b and IgA was analyzed. Furthermore, Z-stack analysis was performed in order to confirm that the CD11b and IgA signals overlapped. Scale bar, 20 µm.

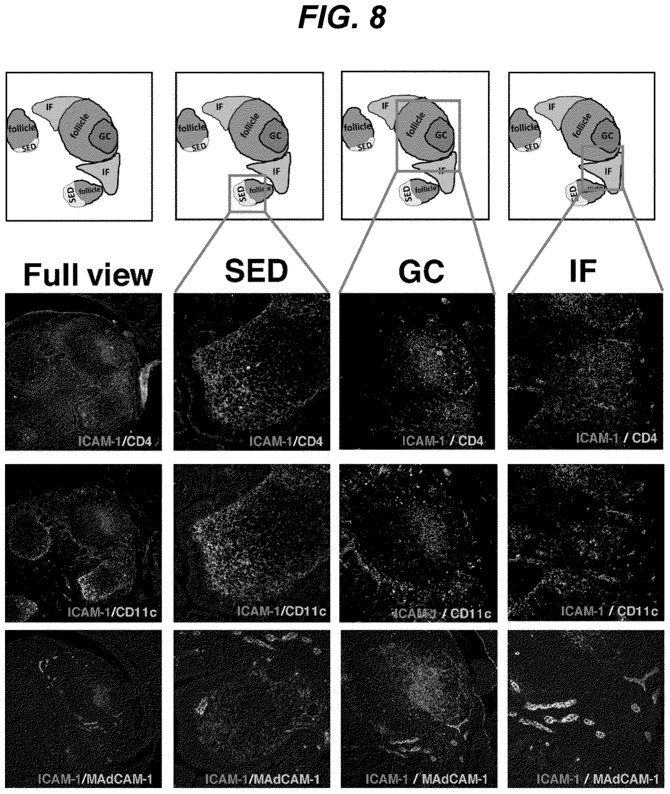

Fig. 8 is a diagram showing the expression of ICAM-1 in HEV. Sections of PP were stained using anti-ICAM-1, anti-CD4 (T cell marker), anti-CD11c (DC marker), and anti-MAdCAM-1 (HEV marker), respectively. SED was identified in a region rich in CD11c⁺ DCs. The IF region was identified in a region enriched with CD4⁺T cells. HEV expressing MAdCAM-1 was found in the IF region. In order to analyze the expression of ICAM-1, the full images and the amplified indicated regions are shown.

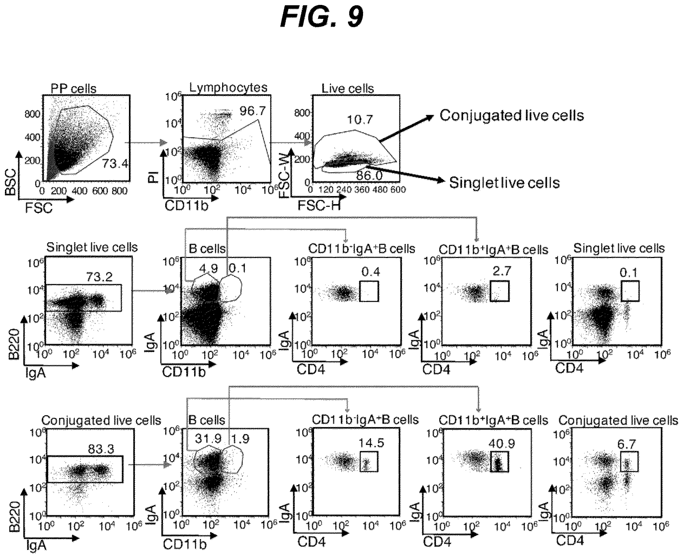

Fig. 9 is a diagram showing the results of analyzing the conjugation between CD11b⁺IgA⁺ B cells and CD4⁺ T. Flow cytometry analysis was performed using 8 to 12-week-old WT Balb/c mice. Dead cells were excluded by PI staining. Single cells and conjugated cells were selected using FSC-H/FSC-W gates. As for the single cells and conjugated cells, conjugation with CD4⁺T cells was analyzed using IgA⁺ cells. As for single live cells and conjugated live cells, B cell gates were selected on B220⁺ cells. The expression of IgA and CD11b in the B220⁺ cells was analyzed. Conjugation of CD4⁺T cells was analyzed in conjugated CD11b⁻IgA⁺ B cells and CD11b⁺IgA⁺ B cells. Single CD11b⁻IgA⁺ B cells and CD11b⁺IgA⁺ B cells were used as negative controls for analyzing the conjugation of the CD4⁺ T cells. Data were obtained from 6 independent experiments.

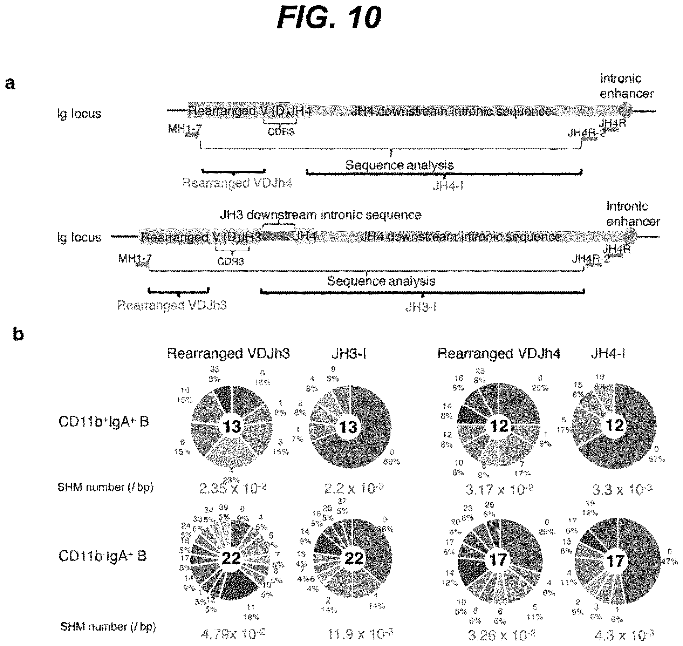

Fig. 10 is a diagram showing that CD11b⁺IgA⁺ B cells with SHM are derived from newly produced activated memory B cells. a, The SHM frequencies in the rearranged VDJ region and the downstream intronic sequences amplified by PCR were analyzed. b, The SHM frequencies were analyzed in the rearranged VDJH₃ and the JH₃ downstream intronic sequence (JH3-I) and the rearranged VDJH₄ and the JH4 downstream intronic sequence (JH4-I) in CD11b⁻IgA⁺ and CD11b⁺IgA⁺ B cells isolated from PPs of 26 WT mice. The pie chart segment size is proportional to the number of sequences with the number of mutations observed in each clone. The number in the center of the pie chart represents the number of clones analyzed.

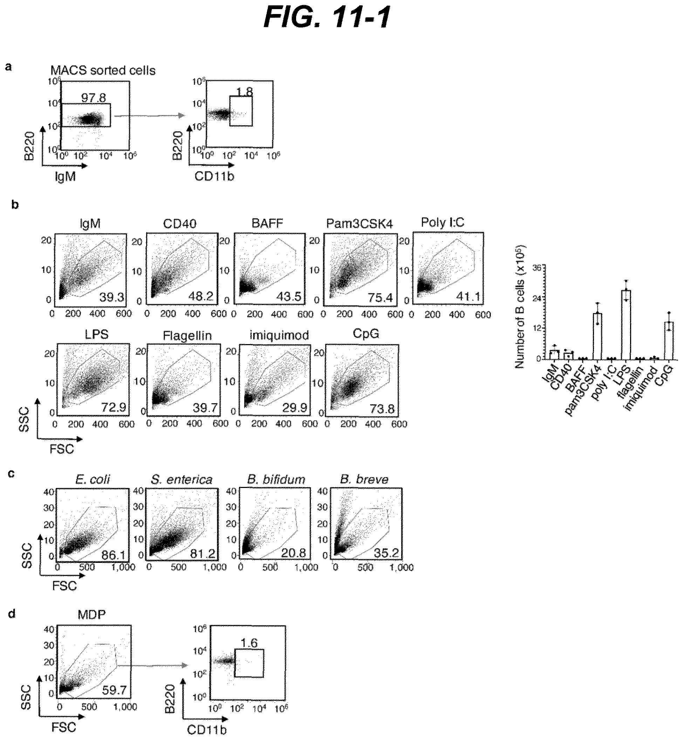

Fig. 11-1 is a diagram showing B cell proliferation and CD11b expression caused by in vitro stimulation, a, Purity of sorted splenic naive B cells and the CD11b expression therein. b, Lymphocyte gates and the numbers (right) of cultured splenic naive B cells. Representative data were obtained from at least 3 independent experiments. c, Lymphocyte gates of the cultured splenic naive B cells were stimulated with the indicated heat-killed bacteria. d, Lymphocyte gate and CD11b expression in cultured splenic naive B cells. Representative data were obtained from 3 independent experiments.

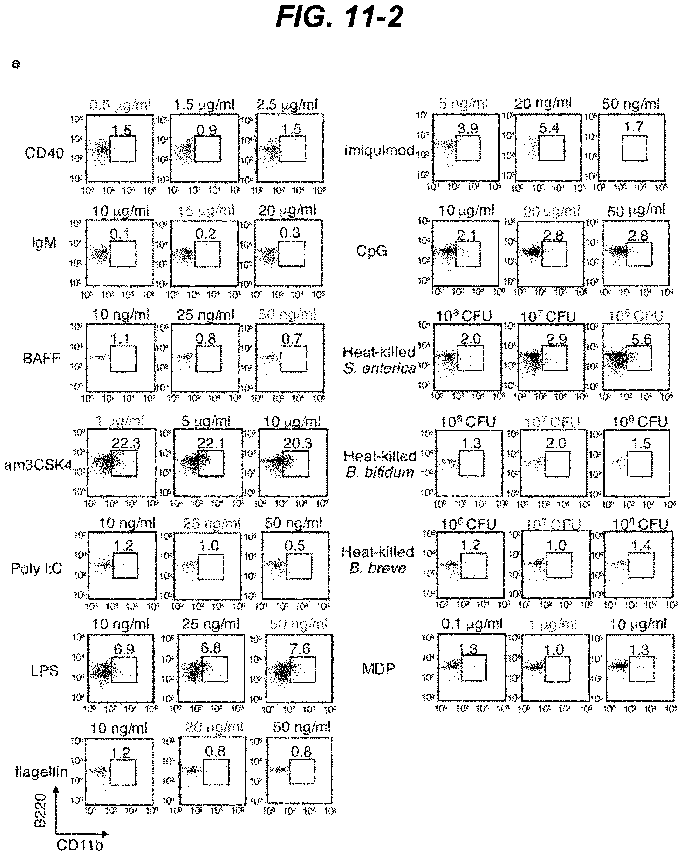

Fig. 11-2 is a diagram showing B cell proliferation and CD11b expression caused by in vitro stimulation, e, CD11b expression in splenic B cells stimulated with the indicated concentration of each stimulus. The conditions selected for the experiments in Figs. 4-1 and 5 are shown in light gray.

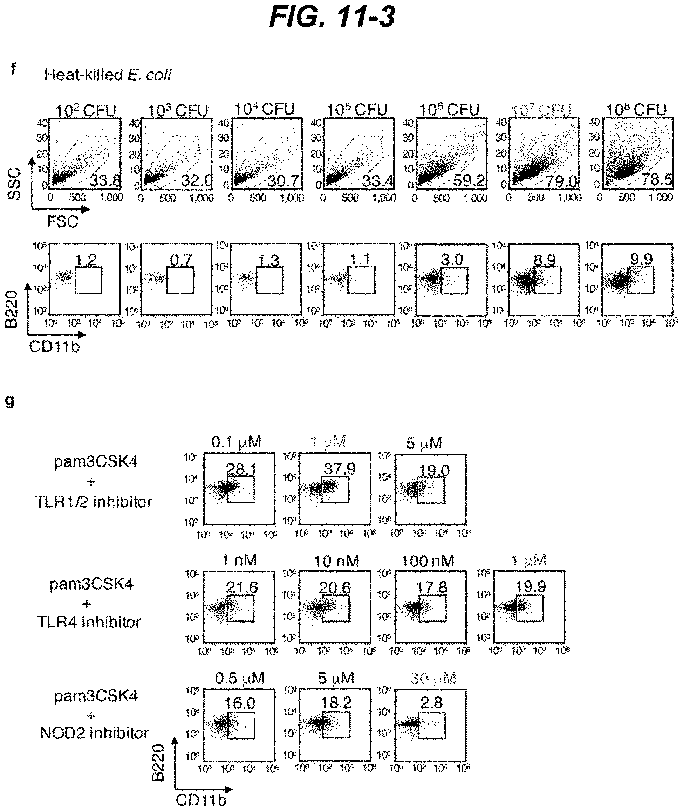

Fig. 11-3 is a diagram showing B cell proliferation and CD11b expression caused by in vitro stimulation, f, CD11b expression in purified splenic B cells stimulated with the indicated concentration of each stimulus. g, CD11b expression in splenic B cells stimulated with pam3CSK4 (1 µg/ml) in the presence of TLR1/2, TLR4, and NOD2 inhibitors at the indicated concentrations. The conditions selected for the experiments in Figs. 4-1 and 5 are shown in light gray.

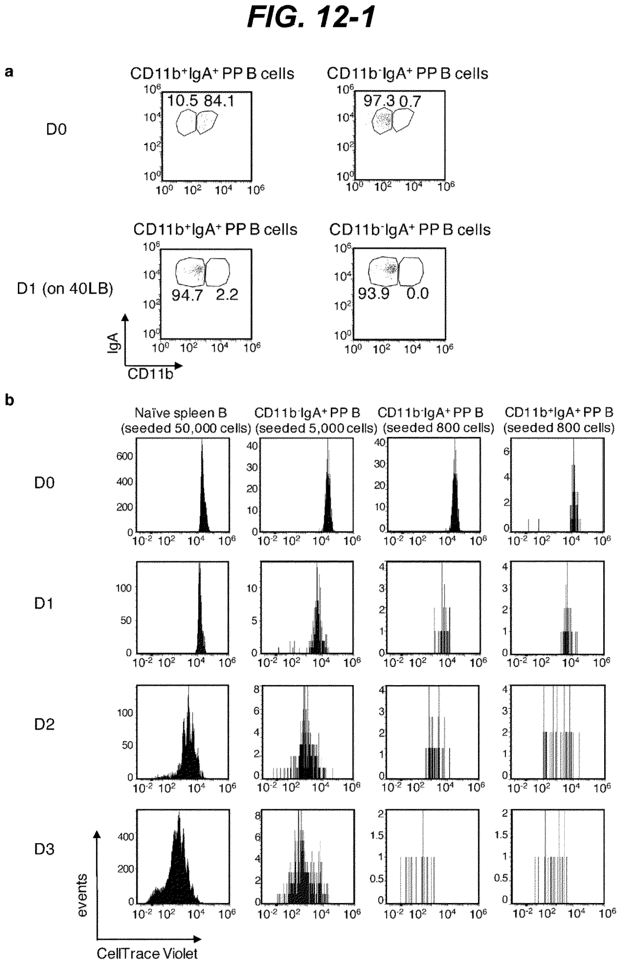

Fig. 12-1 is a diagram showing that isolated CD11b⁺ B cells lost the CD11b expression in an iGB culture system. a, CD11b⁺IgA⁺ and CD11b⁻IgA⁺ PP B cells labeled with CellTrace Violet were cultured in the iGB culture system using IL-21. The CD11b expression was analyzed by flow cytometry on Day 0 and Day 1. b, As positive controls, about 50,000 naive splenic B cells and 5,000 sorted CD11b⁻IgA⁺ B cells labeled with CellTrace Violet were prepared. 800 isolated CD11b⁺IgA⁺ PP B cells and CD11b⁻IgA⁺ PP B cells were prepared to monitor the proliferation thereof. Representative data were obtained from 3 independent experiments.

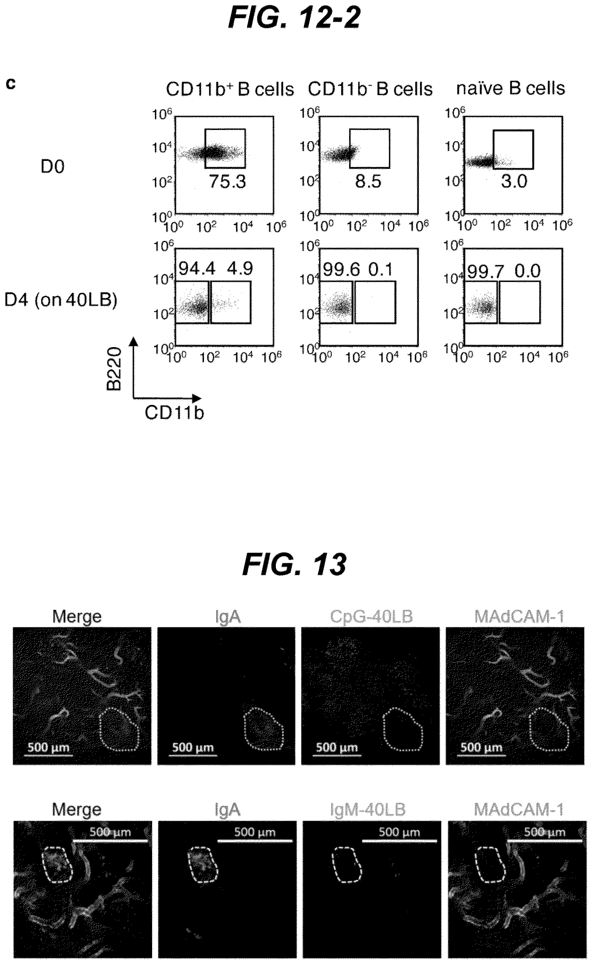

Fig. 12-2 is a diagram showing that isolated CD11b⁺ B cells lost the CD11b expression in an iGB culture system. c, Splenic naive B cells were cultured with pam3CSK4 for 3 days in order to induce the CD11b⁺ B cells. Then, the pam3CSK4-induced CD11b⁺ B cells and CD11b⁻ B cells were isolated. About 1.5 × 10⁵ isolated CD11b⁺ B cells and CD11b⁻ B cells were separately seeded on 40LB cells supplemented with 1 ng/ml IL-4. As a control, negatively sorted splenic naive B cells were used. On D0 and D4, the CD11b expression in the B cells seeded on the 40LB cells was analyzed. Representative data were obtained from 3 independent experiments.
Fig. 13 is a diagram showing that B cells stimulated with IgM-40LB or CpG-40LB do not enter PP GC after being intravenously injected into mice. The indicated iGB cells including CpG-40LB cells (top) and IgM-40LB cells (bottom) were isolated, labeled, and then independently injected intravenously into mice. HEV was identified by intravenously injecting anti-MAdCAM-1 antibody along with the labeled iGB cells. Anti-IgA antibody was directly injected into PPs to stain IgA⁺ cells and identify GCs (dashed circles). Scale bar, 500 um.

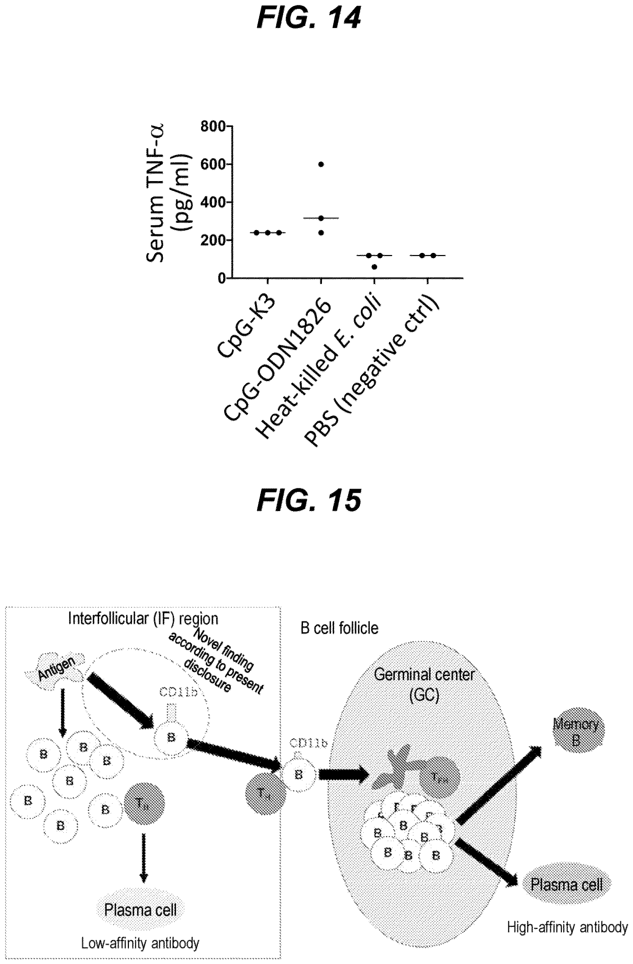

Fig 14 is a diagram showing that an adjuvant composition of the present disclosure did not increase the concentration of serum TNFα at all.
Fig. 15 is a schematic diagram showing that B cells can sense a bacterial antigen and determine their own cell fate into pre-GC cells, independent of DCs and T cells. Description of Embodiments

To identify pre-GC B cells, the inventors focused on mucosal immunity-related immunity-inducing tissues. Since GCs in the mucosal immunity-related immunity-inducing tissues are constitutively activated, pre-GC B cells entering existing GCs can be found at any given time⁷. The inventors considered that integrin CD11b may be a candidate for a marker of pre-GC IgA+ B cells in the mucosal immunity-related immunity-inducing tissues. CD11b, which is 165-kDa-integrin αM, binds to CD18, forming heterodimeric integrin known as macrophage-1 antigen (Mac-1)¹². CD11b is widely known as a marker for myeloid cells and is known to be involved in cell migration and adhesion¹². Furthermore, previous studies have revealed a complex function of CD11b in B cells^{13,14}. Ding et al. revealed that CD11b negatively regulates BCR signaling and maintains tolerance of self-reactive B cells¹³. Kunisawa et al. showed that CD11b is an early stage marker of IgA⁺ PC in mouse intestinal lamina propria (LP), and PP structure is essential for the production of CD11b⁺IgA⁺ PC¹⁵. These studies motivated the inventors to investigate the expression of CD11b in B cells of the mucosal immunity-related immunity-inducing tissues.

In the present disclosure, a small population of IgA⁺ B cells expressing integrin CD11b was identified as pre-GC B cells. These cells were located outside GC and highly expressed irf4. When these CD11b⁺IgA⁺ B cells were injected into mucosal immunity-related immunity-inducing tissues of IgA-Cre/YC3.60^{flox} reporter mice, the injected cells were located around existing GC, instead of directly entering the GC. The cells entered the GC 40 hours later, indicating that the cells were pre-GC IgA⁺ B cells in the mouse mucosal immunity-related immunity-inducing tissues. Furthermore, several bacterial antigens including pam3CSK4, lipopolysaccharide (LPS), and heat-killed Escherichia coli (E. coli) and Salmonella enterica (S. Enterica), but not Bifidobacterium, were found to induce the expression of CD11b in naive B cells in vitro. Expression of CD11b in B cells prior to the interaction with T-B or DC-B allowed intravenously injected B cells to enter existing GCs in mucosal immunity-related immunity-inducing tissues. In this manner, B cells control their own cell fate and become pre-GC B cells, independent of DC activation. In addition, when pam3CSK4 or heat-killed E. coli was orally administered to mice, the number of B cells entering GCs of mucosal immunity-related immunity-inducing tissues increased within 2 days, and mucosal antigen-specific IgA response was enhanced. Inducing CD11b in activated B cells is considered to be a promising marker for pre-GC B cells as well as a useful criterion for selecting an effective mucosal vaccine adjuvant.

### (Composition of the Present Invention)

The composition of the present disclosure includes a substance that induces CD11b expression in B cells and has an effect of inducing migration of activated B cells to a germinal center.

The composition of the present disclosure is expected to exhibit the above effect and can be suitably used in the fields of pharmaceuticals, foods, feeds, and the like. Accordingly, the composition of the present disclosure can be a medicine, a food composition, a food raw material composition, a feed composition, or a feed raw material composition.

### (Substance That Induces CD11b Expression in B Cells)

The substance that induces the CD11b expression in B cells used in the composition of the present disclosure can be obtained by those skilled in the art based on a known method, by referring to Examples presented in the present specification. For example, a candidate compound by which the proportion of CD11b⁺ B cells is increased in B cells after culturing naive splenic B cells isolated from an animal with a candidate substance can be used as the substance that induces the CD11b expression in B cells.

In one aspect, a non-limiting example of the substance that induces the CD11b expression in B cells is bacteria. In one aspect, it is preferable to use gram-negative bacteria. In one aspect, it is preferable to use bacteria of the phylum Proteobacteria or Bacteroidetes. In one aspect, it is preferable to use bacteria of the class γ proteobacteria as the bacteria of the phylum Proteobacteria. In one aspect, it is preferable to use bacteria of the order Enterobacterales. In one aspect, it is more preferable to use Escherichia coli, Salmonella enterica, or Klebsiella pneumoniae. In another aspect, it is preferable to use bacteria of the class Bacteroidia as the bacteria of the phylum Bacteroidetes. In one aspect, it is preferable to use bacteria of the order Bacteroidales. In one aspect, it is preferable to use bacteria of the order Bacteroidales. In one aspect, it is preferable to use Bacteroides vulgatus. As these bacteria of the phylum Proteobacteria or Bacteroidetes, bacteria of various serotypes belonging to a specific species can be used.

In one aspect, the bacteria used as the substance that induces the CD11b expression in B cells are bacteria having a 16S rDNA sequence with a base sequence identity of 90% or more, 910 or more, 92% or more, 93% or more, 94% or more, 95% or more, 96% or more, 98% or more, 99% or more, or 99.5% or more with the 16S rDNA sequence of a representative serotype of Escherichia coli, Salmonella enterica, Klebsiella pneumoniae, or Bacteroides vulgatus.

In one aspect, the bacteria used as the substance that induces the CD11b expression in B cells are bacteria having a 16S rDNA sequence with a base sequence identity of 90% or more, 910 or more, 92% or more, 93% or more, 94% or more, 95% or more, 96% or more, 98% or more, 99% or more, or 99.5% or more with the following 16S rDNA sequences.

Escherichia coli 16S rDNA sequence: DNA sequence encoding 16S ribosomal RNA of GenBank registration number CP003278.1 (https://www.ncbi.nlm.nih.gov/nuccore/CP017100) (sequence consisting of the bases at positions 225107 to 226662) .

Salmonella enterica 16S rDNA sequence: DNA sequence encoding 16S ribosomal RNA of GenBank registration number CP003278.1 (https://www.ncbi.nlm.nih.gov/nuccore/CP003278.1) (complementary strand of the sequence containing the bases at positions 287486 to 289015 or complementary strand of the sequence containing the bases at positions 3396200 to 3397729) .

Klebsiella pneumoniae 16S rDNA sequence: DNA sequence encoding 16S ribosomal RNA of GenBank registration number CP018306.1 (https://www.ncbi.nlm.nih.gov/nuccore/CP018306) (complementary strand of the sequence containing the bases at positions 503553 to 505106, complementary strand of the sequence containing the bases at positions 1312903 to 1314456, sequence containing the bases at positions 1818027 to 1819580 and 1926864 to 1928417, sequence containing the bases at positions 2018746 to 2020299, sequence containing the bases at positions 2444117 to 2445670, or sequence containing the bases at positions 2799428 to 2800981).

Bacteroides vulgatus 16S rDNA sequence: DNA sequence encoding 16S ribosomal RNA of GenBank registration number AP025232.1 (https://www.ncbi.nlm.nih.gov/nuccore/AP025232) (complementary strand of the sequence containing the bases at positions 294690 to 296214, complementary strand of the sequence containing the bases at positions 626688 to 628212, sequence containing the bases at positions 1871179 to 1872703, sequence containing the bases at positions 1957949 to 1959473, sequence containing the bases at positions 2375844 to 2377368, complementary strand of the sequence containing the bases at positions 4187249 to 4188773, or complementary strand of the sequence containing the bases at positions 4496890 to 4498414).

The composition of the present disclosure has an effect of inducing migration of activated B cells to a germinal center. In one aspect, the composition of the present disclosure has an effect of inducing the migration of activated B cells to a germinal center of a mucosal immunity-related immunity-inducing tissue. The mucosal immunity-related immunity-inducing tissue is not particularly limited and includes mucosal immunity-related immunity-inducing tissues that lead to affinity maturation of activated B cells. For example, the mucosal immunity-related immunity-inducing tissues include mucosal immunity-related immunity-inducing tissues in the gastrointestinal tract (in the intestine, oral cavity, or the like), nasal cavity, and the like. In one aspect, the mucosal immunity-related immunity-inducing tissue is Peyer's patch, and the composition of the present disclosure has an effect of inducing the migration of activated B cells to a germinal center of Peyer's patch.

In a case where bacteria are used as the substance that induces the CD11b expression in B cells, heat-sterilized bacteria or live bacteria can be used.

The amount of the substance that induces the CD11b expression in B cells in the composition of the present disclosure is not particularly limited, as long as the effect of inducing the migration of activated B cells to a germinal center can be exhibited. For example, the substance that induces the CD11b expression in B cells is mixed into the composition in a range of 0.001 to 99.99 weight%.

### (Pharmaceutical Composition)

In one aspect, the composition of the present disclosure including the substance that induces the CD11b expression in B cells is provided as a pharmaceutical composition. The pharmaceutical composition of the present disclosure is typically used to stimulate immunity. In one aspect, the pharmaceutical composition of the present disclosure is used to provide antigen-specific defense in the mucosal tissue or distal mucosal tissue by inducing activation of migration of IgA-producing B cells to a germinal center, affinity maturation, and high-affinity antibody production. In one aspect, the pharmaceutical composition of the present disclosure is used to activate the migration of IgA-producing B cells to a germinal center of a mucosal immunity-related immunity-inducing tissue.

The mucosal immunity-related immunity-inducing tissue is not particularly limited and includes mucosal immunity-related immunity-inducing tissues that lead to affinity maturation of activated B cells. For example, the mucosal immunity-related immunity-inducing tissues include mucosal immunity-related immunity-inducing tissues in the gastrointestinal tract (in the intestine, oral cavity, or the like), nasal cavity, and the like. In one aspect, the mucosal immunity-related immunity-inducing tissue is Peyer's patch, and the composition of the present disclosure is used to induce the migration of activated B cells to a germinal center of Peyer's patch.

The antigen targeted by the immunity stimulated by the pharmaceutical composition of the present disclosure may an antigen already existing in in vivo mucosal tissue or may be administered together with the pharmaceutical composition of the present disclosure. In one aspect, the pharmaceutical composition of the present disclosure is used to stimulate immunity. In one aspect, the pharmaceutical composition of the present disclosure is preferably used to stimulate mucosal immunity, and in one aspect, the pharmaceutical composition of the present disclosure is used to stimulate immunity in the gastrointestinal tract (in intestine, oral cavity, or the like) or in the nasal cavity.

In one aspect, the pharmaceutical composition of the present disclosure is provided as an adjuvant composition not comprising an antigen. In one aspect, the pharmaceutical composition of the present disclosure is administered together with an antigen. In one aspect, the pharmaceutical composition of the present disclosure is administered simultaneously with an antigen. In one aspect, the pharmaceutical composition of the present disclosure is administered separately from an antigen. In a case where the pharmaceutical composition of the present disclosure is administered separately from an antigen, each administration interval can be suitably selected. For example, the duration between each administration can be 5 minutes, 10 minutes, 20 minutes, 30 minutes, 1 hour, 2 hours, 3 hours, 4 hours, 5 hours, 6 hours, 7 hours, 8 hours, 9 hours, 10 hours, 11 hours, 12 hours, 24 hours, 1 day, 2 days, 3 days, 4 days, 5 days, 6 days, 7 days, or the like, but the duration is not limited thereto. The frequencies of administering the pharmaceutical composition of the present disclosure and an antigen may be the same as or different from each other. The forms of the pharmaceutical composition of the present disclosure and a composition comprising an antigen may be the same as or different from each other. For example, the pharmaceutical composition of the present disclosure may be combined with a composition comprising an antigen in a food form. In one aspect, the pharmaceutical composition of the present disclosure is provided as a vaccine composition comprising an antigen. The antigen is not particularly limited, and, for example, a viral, bacterial, fungal, or parasitic antigen is selected.

In one aspect, the pharmaceutical composition of the present disclosure is used for treatment of a subject with a disease, disorder, or condition which is treated by activation of the migration of activated B cells to a germinal center. In one aspect, the composition of the present disclosure is used for maintaining or improving health with immunostimulation, maintaining or improving mucosal flora (for example, intestinal flora), or suppression of infection (for example, suppression of viral, bacterial, fungal, or parasitic infection) through immunostimulation (for example, mucosal immunostimulation and intestinal immunostimulation).

Therefore, in one aspect, the present disclosure provides a pharmaceutical composition for inducing migration of activated B cells to a germinal center, the pharmaceutical composition including the substance that induces the CD11b expression in B cells.

Furthermore, in one aspect, the present disclosure provides a pharmaceutical composition for treatment of a subject with a disease, disorder, or condition which is treated by activation of the migration of activated B cells to a germinal center, the pharmaceutical composition including the substance that induces the CD11b expression in B cells.

In addition, in one aspect, the present disclosure provides a pharmaceutical composition for immunostimulation, the pharmaceutical composition including the substance that induces the CD11b expression in B cells. The stimulation of mucosal immunity includes stimulation of gastrointestinal immunity (for example, intestinal immunity and oral immunity) and nasal immunity, but the stimulation of mucosal immunity is not limited thereto.

In addition, in one aspect, the present disclosure provides a pharmaceutical composition for stimulation of antigen-specific immunity in a mucosa, the pharmaceutical composition including the substance that induces the CD11b expression in B cells. The mucosa includes gastrointestinal mucosa (for example, intestinal mucosa and oral mucosa) and nasal mucosa, but the mucosa is not limited thereto.

In one aspect, the immunostimulation includes the effect of maintaining or improving health involving immunostimulation (for example, mucosal immunostimulation and intestinal immunostimulation), the effect of maintaining or improving health involving maintenance or improvement of mucosal flora (for example, intestinal flora), and treatment of an infectious disease (for example, treatment of viral infection, bacterial infection, fungal infection, or parasitic infection), but the immunostimulation is not limited thereto.

Furthermore, in one aspect, the subject in need of the stimulation of antigen-specific immunity in the mucosa is a subject in need of immunostimulation (for example, mucosal immunostimulation and intestinal immunostimulation), a subject in need of maintaining or improving health involving immunostimulation, a subject in need of maintaining or improving mucosal flora (for example, intestinal flora), a subject in need of maintaining or improving health involving mucosal flora, or a subject in need of treatment of an infectious disease (for example, treatment of viral infection, bacterial infection, fungal infection, or parasitic infection).

In addition, in one aspect, the present disclosure provides a composition for maintaining or improving health through immunostimulation, the composition including the substance that induces the CD11b expression in B cells.

Moreover, in one aspect, the present disclosure provides a composition for maintaining or improving mucosal flora (for example, intestinal flora) through immunostimulation, the composition including the substance that induces the CD11b expression in B cells.

Furthermore, in one aspect, the present disclosure provides a pharmaceutical composition for treating an infectious disease (for example, treatment of viral infection, bacterial infection, fungal infection, or parasitic infection), the pharmaceutical composition including the substance that induces the CD11b expression in B cells.

The term "treatment" in the present disclosure has the general meaning in the art, and includes preventing the occurrence of any disorder or disease state for the purpose of ameliorating, eradicating, or curing the disorder or disease state, or administering the composition of the present disclosure to a subject for the purpose of delaying (preventing) the occurrence of the disorder or disease state or for the purpose of preventing recurrence of the disorder or disease state or reducing the possibility of the recurrence (suppression of recurrence).

In one aspect, the pharmaceutical composition of the present disclosure is a pharmaceutical composition for stimulating mucosal immunity. In one aspect, the pharmaceutical composition of the present disclosure is a composition used as a vaccine adjuvant which is administered mucosally. In one aspect, the pharmaceutical composition of the present disclosure is a composition used to promote the expression of a high-affinity mucosal IgA antibody.

The pharmaceutical composition of the present disclosure may be any pharmaceutical composition as long as it includes the substance that induces the CD11b expression in B cells. For example, the content of the substance according to the present invention that induces the CD11b expression in B cells in 100 weight% of the pharmaceutical composition can be set as appropriate within the range of 0.001 to 99.99 weight%, taking into account the type of target disease, the dosage form, the administration method, the subject of the administration, the degree of a symptom in the subject of the administration, the degree of effect exerted by the administration, and the like. In one aspect, the content of the substance according to the present invention that Induces the CD11b expression in B cells in 100 weight% of the pharmaceutical composition may be 0.001 to 99.99 weight%, 0.01 to 99.99 weight%, 0.1 to 99.99 weight%, 0.2 to 99.99 weight%, 0.3 to 99.99 weight%, 0.4 to 99.99 weight%, 0.5 to 99.99 weight%, 0.6 to 99.99 weight%, 0.7 to 99.99 weight%, 0.8 to 99.99 weight%, 0.9 to 99.99 weight%, 1.0 to 99.99 weight%, 2.0 to 99.99 weight%, 3.0 to 99.99 weight%, 4.0 to 99.99 weight%, 5.0 to 99.99 weight%, 6.0 to 99.99 weight%, 7.0 to 99.99 weight%, 8.0 to 99.99 weight%, 9.0 to 99.99 weight%, 10 to 99.99 weight%, 20 to 99.99 weight%, 30 to 99.99 weight%, 40 to 99.99 weight%, 50 to 99.99 weight%, 60 to 99.99 weight%, 70 to 99.99 weight%, 80 to 99.99 weight%, or 90 to 99.99 weight%. In one aspect, the content of the substance according to the present invention that Induces the CD11b expression in B cells in 100 weight% of the pharmaceutical composition may be 0.001 to 90 weight%, 0.001 to 80 weight%, 0.001 to 70 weight%, 0.001 to 60 weight%, 0.001 to 50 weight%, 0.001 to 40 weight%, 0.001 to 30 weight%, 0.001 to 20 weight%, 0.001 to 10 weight%, 0.001 to 9.0 weight%, 0.001 to 8.0 weight%, 0.001 to 7.0 weight%, 0.001 to 6.0 weight%, 0.001 to 5.0 weight%, 0.001 to 4.0 weight%, 0.001 to 3.0 weight%, 0.001 to 2.0 weight%, or 0.001 to 1.0 weight%. In one aspect, the content of the substance according to the present invention that induces the CD11b expression in B cells in 100 weight% of the pharmaceutical composition is preferably 1.0 to 80 weight%, 5.0 to 70 weight%, 10 to 60 weight%, 20 to 50 weight%, or 30 to 40 weight%, but the content is not limited thereto.

The term "effective dose" in the present disclosure refers to the amount of the substance that induces CD11b expression in B cells in the present disclosure that can induce the migration of activated B cells to a germinal center in a living body.

The pharmaceutical composition according to the present disclosure may contain a pharmaceutically acceptable carrier or additive along with the substance that induces the CD11b expression in B cells. The pharmaceutically acceptable carrier or additive means any carrier, diluent, excipient, suspending agent, lubricant, adjuvant, vehicle, delivery system, emulsifier, disintegrant, absorbent, preservative, surfactant, colorant, flavor, or sweetener, and any known pharmaceutically acceptable carrier or additive may be adopted.

The subject of administration is not particularly limited, but non-limiting examples include, for example, mammals such as a human, a mouse, a rat, a guinea pig, a rabbit, a hamster, a dog, a cat, a weasel, a cow, a pig, a horse, a deer, a wild boar, a sheep, and a goat, birds including poultry such as a chicken, a duck, a turkey, and a quail, reptiles such as a snake and a turtle, amphibians such as a frog, fishes such as a salmonid fish, species of Seriola and cobia, a freshwater trout, a sweetfish, species of sea bass, sea bream, and Argyrosomus japonicus, a flounder, a fugu, a young yellowtail, an allied kingfish, a bluefin tuna, a jack mackerel, a striped jack, a tiger puffer, a yellowtail amberjack, a striped beakfish, a thread-sail filefish, a darkbanded rockfish, a scorpionfish, a black sea bream, a black rockfish, a perch, a red seabream, a chicken grunt, a girella, a chub mackerel, a seven band grouper, a longtooth grouper, an eel, a catfish, a loach, a sturgeon, a tropical fish, a tilapia, a pangasius, a black carp, a bighead carp, a silver carp, a grass carp, and a carp, a bivalve (an oyster, a scallop, an asari clam, a mussel, or an Akoya pearl oyster), an abalone, a shrimp, and seaweed.

The dosage and administration method for the pharmaceutical composition of the present disclosure are appropriately selected depending on the type of disease affecting the individual to be administered with the pharmaceutical composition, sex, species, age, general condition, severity of the disease, desired degree of effect, and the like. Normally, the dosage may be appropriately set within the range of 0.001 to 100 mg/kg/day. For example, in a case where bacteria are used as the substance that induces the CD11b expression in B cells, based on the bacterial load, the dosage can be appropriately set within the range of 10⁶ CFU/time or more, 10⁷ CFU/time or more, 10⁸ CFU/time or more, 10⁹ CFU/time or more, 10¹⁰ CFU/ time or more, or the like for, for example, a mouse weighing 20 g, or within the range of 3 × 10⁹ CFU/time or more, 3 × 10¹⁰ CFU/time or more, 3 × 10¹¹ CFU/time or more, 3 × 10¹² CFU/time or more, 3 × 10¹³CFU/time or more, or the like for a human weighing 60 kg, but the dosage is not limited thereto. The above-described dosage per time can be administered, for example, once to three times a day. In one aspect, in the case of using in a human weighing 60 kg, the administration is preferably performed at a dosage within the range of 3 × 10¹⁰ CFU/day to 3 × 10¹³ CFU/day, 3 × 10¹¹ CFU/day to 3 × 10¹²CFU/day, or the like, but the dosage is not limited thereto.

The above-described amount of the pharmaceutical composition according to the present disclosure may be administered once a day or may be administered in several divided doses. Furthermore, the administration interval may be daily, every other day, every week, every other week, every 2 or 3 weeks, every month, every other month, or every 2 or 3 months, as long as the administration has a therapeutic effect on the disease.

Although the administration method for the pharmaceutical composition of the present disclosure is not particularly limited, it is preferable to directly administer the pharmaceutical composition to a mucosal tissue such as the gastrointestinal tract, and, as such transmucosal administration, for example, oral administration, transnasal administration, transvaginal administration, enteral administration, or the like can be used. The composition of the present disclosure which is administered through the mucosae of the various organs induces affinity maturation of IgA-secreting B cells and production of high-affinity IgA antibodies in mucosal immunity-related immunity-inducing tissues, thus leading to antigen-specific defense in tissues such as a mucosal tissue and a distal mucosal tissue to which the composition is administered.

Here, the enteral administration is not limited to administration via the anus and also includes, for example, administration via a tube or the like inserted into the gastrointestinal tract from outside of an individual, as in the case of gastrostomy. The position of the insertion of the tube in the gastrointestinal tract is not limited to the intestine, and examples of the position include esophagus, stomach, small intestine (including duodenum, jejunum, ileum, or the like) and large intestine (including cecum, colon, rectum, or the like). Furthermore, it is also possible to use the composition of the present disclosure as, for example, a gut lavage fluid, by mixing the composition together with a known component.

In one aspect, the pharmaceutical composition of the present disclosure can be used in combination with another therapeutic agent, for example, a vaccine containing an antigen, to the extent that does not impair the function of the pharmaceutical composition.

In a case where a plurality of the pharmaceutical compositions of the present disclosure are used in combination, or the pharmaceutical composition of the present disclosure is used in combination with another therapeutic drug, these agents may be contained in a single formulation or in separate formulations. In a case where these agents are provided in separate formulations, the formulations may be provided in the form of a kit containing a combination of the formulations, or each formulation may be provided as a single agent for combination with other formulations. These formulations may be administered simultaneously or sequentially. In one aspect, the pharmaceutical composition of the present disclosure is provided in the form of a raw material composition of the finally provided pharmaceutical composition.

### (Food Composition)

In one aspect, the composition of the present disclosure including the substance that induces the CD11b expression in B cells is provided as a food composition. The food composition is a composition obtained by suitably using the immunostimulatory composition of the present disclosure exclusively in the field of food.

The form of the food composition of the present disclosure is not particularly limited, and for example, the food composition can be provided as a food or beverage such as a supplement, a solid food, a liquid food, and a drink. The food composition of the present disclosure can be provided as a special purpose food such as a food for specified health use, a food with functional claims, or a food with nutrient function claims. The food composition of the present disclosure can be provided as a food with health claims, a food for patients, a dairy product, fermented milk, infant formula, a lactic acid bacteria beverage, an acidic beverage, yogurt, cheese, bread, biscuits, crackers, pizza crust, infant formula, a liquid food, a food for patients, a nutritional food, a frozen food, a food composition, a processed food, or other commercially available foods.

In addition to general foods, the food composition of the present disclosure includes food for specified health use including qualified food for specified health use, a nutritional supplement, a functional food, a food for patients, and the like. In one aspect, the food composition of the present disclosure can be provided as, for example, a food composition labeled with immunostimulation, mucosal immunostimulation, intestinal immunostimulation, maintenance or improvement of health, maintenance or improvement of mucosal flora, maintenance or improvement of intestinal flora, viral infection control, bacterial infection control, fungal infection control, or similar indications, or a food composition for such uses.

In one aspect, the food composition of the present disclosure is administered together with an antigen. In one aspect, the food composition of the present disclosure is administered simultaneously with an antigen. In one aspect, the food composition of the present disclosure is administered separately from an antigen. In a case where the food composition of the present disclosure is administered separately from an antigen, each administration interval can be suitably selected. For example, the duration between each administration can be 5 minutes, 10 minutes, 20 minutes, 30 minutes, 1 hour, 2 hours, 3 hours, 4 hours, 5 hours, 6 hours, 7 hours, 8 hours, 9 hours, 10 hours, 11 hours, 12 hours, 24 hours, 1 day, 2 days, 3 days, 4 days, 5 days, 6 days, 7 days, or the like, but the duration is not limited thereto. The frequencies of administering the food composition of the present disclosure and an antigen may be the same as or different from each other. The forms of the food composition of the present disclosure and a composition comprising an antigen may be the same as or different from each other. For example, the food composition of the present disclosure may be combined with a pharmaceutical composition containing an antigen. In one aspect, the food composition of the present disclosure is provided as a composition comprising an antigen. The antigen is not particularly limited, and, for example, a viral, bacterial, fungal, or parasitic antigen is selected.

Specific forms of the food composition of the present disclosure is not particularly limited, and examples thereof can include beverages such as soft drink, carbonated drink, energy drink, fruit drink, lactic acid drink, and milk beverage; frozen desserts such as ice cream, ice sorbet, and shaved ice; confectionaries such as sweets, candies, gum, chocolate, tablet candies, snacks, biscuits, jellies, jam, cream, and baked goods; noodles such as soba noodles, wheat noodles, vermicelli, Chinese noodles, and instant noodles; aquatic and livestock processed foods such as boiled fish paste, ham, and sausage; dairy products such as processed milk and fermented milk; oils and fats and oil and fat processed foods such as salad oil, tempura oil, margarine, mayonnaise, shortening, whipped cream, and dressing; seasonings such as sauce and gravy; soup, stew, salad, daily dish, rice seasoning, pickle, bread, and cereal. In addition, in the cases of food for specified health use, nutritional supplement, functional food, and the like, the food composition can be provided in the form such as, for example, powder, granules, capsules, lozenges, tablets, and syrup.

In one embodiment, the composition of the present disclosure is provided as a food material. The form of the food material of the present disclosure is not particularly limited and is suitably set as, for example, liquid, paste, powder, solid, or the like, according to the type of food to be used. The food material of the present disclosure may be further subjected to processing such as crushing, pulverizing, and granulating as necessary to form the food material into a desired form.

The food composition of the present disclosure may be any food composition as long as it includes the substance according to the present disclosure that induces the CD11b expression in B cells. For example, the content of the antibody according to the present invention in 100 weight% of the composition can be set as appropriate within the range of 0.001 to 99.99 weight%, taking into account the degree of effect exerted by the composition or the like. In one aspect, the content of the substance according to the present invention that Induces the CD11b expression in B cells in 100 weight% of the food composition may be 0.001 to 99.99 weight%, 0.01 to 99.99 weight%, 0.1 to 99.99 weight%, 0.2 to 99.99 weight%, 0.3 to 99.99 weight%, 0.4 to 99.99 weight%, 0.5 to 99.99 weight%, 0.6 to 99.99 weight%, 0.7 to 99.99 weight%, 0.8 to 99.99 weight%, 0.9 to 99.99 weight%, 1.0 to 99.99 weight%, 2.0 to 99.99 weight%, 3.0 to 99.99 weight%, 4.0 to 99.99 weight%, 5.0 to 99.99 weight%, 6.0 to 99.99 weight%, 7.0 to 99.99 weight%, 8.0 to 99.99 weight%, 9.0 to 99.99 weight%, 10 to 99.99 weight%, 20 to 99.99 weight%, 30 to 99.99 weight%, 40 to 99.99 weight%, 50 to 99.99 weight%, 60 to 99.99 weight%, 70 to 99.99 weight%, 80 to 99.99 weight%, or 90 to 99.99 weight%. In one aspect, the content of the substance according to the present invention that Induces the CD11b expression in B cells in 100 weight% of the food composition may be 0.001 to 90 weight%, 0.001 to 80 weight%, 0.001 to 70 weight%, 0.001 to 60 weight%, 0.001 to 50 weight%, 0.001 to 40 weight%, 0.001 to 30 weight%, 0.001 to 20 weight%, 0.001 to 10 weight%, 0.001 to 9.0 weight%, 0.001 to 8.0 weight%, 0.001 to 7.0 weight%, 0.001 to 6.0 weight%, 0.001 to 5.0 weight%, 0.001 to 4.0 weight%, 0.001 to 3.0 weight%, 0.001 to 2.0 weight%, or 0.001 to 1.0 weight%. In one aspect, the content of the substance according to the present invention that induces the CD11b expression in B cells in 100 weight% of the food composition is preferably 1.0 to 80 weight%, 5.0 to 70 weight%, 10 to 60 weight%, 20 to 50 weight%, or 30 to 40 weight%, but the content is not limited thereto.

In one aspect, the composition of the present disclosure is provided as a food additive added when the above food is consumed or as a raw material composition for producing the above food or food additive.

### (Feed Composition)

In one aspect, the composition of the present disclosure including the substance that induces the CD11b expression in B cells is provided as a feed composition. The feed composition is a composition obtained by using the feed composition of the present disclosure exclusively in the field of feed.

Specific forms of the above-described feed composition are not particularly limited, and, for example, the feed composition may be prepared by mixing with an ordinary feed or by mixing with an ingredient that can be mixed with an ordinary feed as necessary, as long as the effect exerted by the feed composition according to the present disclosure described above is not impaired, or the feed composition may be used as the feed per se.

The feed composition of the present disclosure can be provided as, for example, a feed composition labeled with immunostimulation, mucosal immunostimulation, intestinal immunostimulation, maintenance or improvement of mucosal flora, maintenance or improvement of intestinal flora, maintenance or improvement of health, maintenance or improvement of intestinal flora, viral infection control, bacterial infection control, fungal infection control, or similar indications, or a feed composition for such uses.

In one aspect, the feed composition of the present disclosure is administered together with an antigen. In one aspect, the feed composition of the present disclosure is administered simultaneously with an antigen. In one aspect, the feed composition of the present disclosure is administered separately from an antigen. In a case where the feed composition of the present disclosure is administered separately from an antigen, each administration interval can be suitably selected. For example, the duration between each administration can be 5 minutes, 10 minutes, 20 minutes, 30 minutes, 1 hour, 2 hours, 3 hours, 4 hours, 5 hours, 6 hours, 7 hours, 8 hours, 9 hours, 10 hours, 11 hours, 12 hours, 24 hours, 1 day, 2 days, 3 days, 4 days, 5 days, 6 days, 7 days, or the like, but the duration is not limited thereto. The frequencies of administering the feed composition of the present disclosure and an antigen may be the same as or different from each other. The forms of the feed composition of the present disclosure and a composition comprising an antigen may be the same as or different from each other. For example, the feed composition of the present disclosure may be combined with a pharmaceutical composition comprising an antigen. In one aspect, the feed composition of the present disclosure is provided as a composition comprising an antigen. The antigen is not particularly limited, and, for example, a viral, bacterial, fungal, or parasitic antigen is selected.

The feed composition of the present disclosure may be any feed composition as long as it includes the substance according to the present disclosure that induces the CD11b expression in B cells. For example, the content of the antibody according to the present invention in 100 weight% of the composition can be set as appropriate within the range of 0.001 to 99.99 weight%, taking into account the degree of effect exerted by the composition or the like. In one aspect, the content of the substance according to the present invention that Induces the CD11b expression in B cells in 100 weight% of the feed composition may be 0.001 to 99.99 weight%, 0.01 to 99.99 weight%, 0.1 to 99.99 weight%, 0.2 to 99.99 weight%, 0.3 to 99.99 weight%, 0.4 to 99.99 weight%, 0.5 to 99.99 weight%, 0.6 to 99.99 weight%, 0.7 to 99.99 weight%, 0.8 to 99.99 weight%, 0.9 to 99.99 weight%, 1.0 to 99.99 weight%, 2.0 to 99.99 weight%, 3.0 to 99.99 weight%, 4.0 to 99.99 weight%, 5.0 to 99.99 weight%, 6.0 to 99.99 weight%, 7.0 to 99.99 weight%, 8.0 to 99.99 weight%, 9.0 to 99.99 weight%, 10 to 99.99 weight%, 20 to 99.99 weight%, 30 to 99.99 weight%, 40 to 99.99 weight%, 50 to 99.99 weight%, 60 to 99.99 weight%, 70 to 99.99 weight%, 80 to 99.99 weight%, or 90 to 99.99 weight%. In one aspect, the content of the substance according to the present invention that Induces the CD11b expression in B cells in 100 weight% of the feed composition may be 0.001 to 90 weight%, 0.001 to 80 weight%, 0.001 to 70 weight%, 0.001 to 60 weight%, 0.001 to 50 weight%, 0.001 to 40 weight%, 0.001 to 30 weight%, 0.001 to 20 weight%, 0.001 to 10 weight%, 0.001 to 9.0 weight%, 0.001 to 8.0 weight%, 0.001 to 7.0 weight%, 0.001 to 6.0 weight%, 0.001 to 5.0 weight%, 0.001 to 4.0 weight%, 0.001 to 3.0 weight%, 0.001 to 2.0 weight%, or 0.001 to 1.0 weight%. In one aspect, the content of the substance according to the present invention that induces the CD11b expression in B cells in 100 weight% of the feed composition is preferably 1.0 to 80 weight%, 5.0 to 70 weight%, 10 to 60 weight%, 20 to 50 weight%, or 30 to 40 weight%, but the content is not limited thereto.

In one aspect, the composition of the present disclosure is provided as a feed additive added when the above feed is consumed or as a raw material composition for producing the above feed or feed additive.

The subject of administering the food composition or feed composition of the present disclosure is not particularly limited, but non-limiting examples include, for example, mammals such as a human, a mouse, a rat, a guinea pig, a rabbit, a hamster, a dog, a cat, a weasel, a cow, a pig, a horse, a deer, a wild boar, a sheep, and a goat, birds including poultry such as a chicken, a duck, a turkey, and a quail, reptiles such as a snake and a turtle, amphibians such as a frog, fishes such as a salmonid fish, species of Seriola and cobia, a freshwater trout, a sweetfish, species of sea bass, sea bream, and Argyrosomus japonicus, a flounder, a fugu, a young yellowtail, an allied kingfish, a bluefin tuna, a jack mackerel, a striped jack, a tiger puffer, a yellowtail amberjack, a striped beakfish, a thread-sail filefish, a darkbanded rockfish, a scorpionfish, a black sea bream, a black rockfish, a perch, a red seabream, a chicken grunt, a girella, a chub mackerel, a seven band grouper, a longtooth grouper, an eel, a catfish, a loach, a sturgeon, a tropical fish, a tilapia, a pangasius, a black carp, a bighead carp, a silver carp, a grass carp, and a carp, a bivalve (an oyster, a scallop, an asari clam, a mussel, or an Akoya pearl oyster), an abalone, a shrimp, and seaweed.

### (Method for Treatment or Maintenance/Improvement of Health)

In one aspect, the present disclosure includes a treatment method or health maintenance or improvement method using the substance that induces CD11b expression in B cells and the pharmaceutical composition, food composition, or feed composition comprising the substance. In the present disclosure, the treatment or health maintenance or improvement method includes administering, to a subject, the substance of the present disclosure that induces CD11b expression in B cells or the "composition", "pharmaceutical composition", "food composition", or "feed composition" of the present disclosure. The specific disorder or disease to be prevented or treated, subject of the administration, administration method, and dosage may also be as described in the above "composition", "pharmaceutical composition", "food composition", or "feed composition". Furthermore, the effect of maintaining or improving health which is obtained by the method of the present disclosure is not particularly limited, as long as the effect leads to the migration of activated B cells to a germinal center.

In one aspect, the present disclosure provides a method for causing the migration of activated B cells to a germinal center in a subject in need of activation of the migration of activated B cells to a germinal center, the method including administering a composition comprising a substance that induces CD11b expression in B cells to the subject.

In addition, in one aspect, the present disclosure provides a method for treating a subject with a disease, disorder, or condition which is treated by activation of migration of activated B cells to a germinal center, the method including administering a composition including a substance that induces CD11b expression in B cells to the subject.

Moreover, in one aspect, the present disclosure provides a method for causing immunostimulation in a subject in need of the immunostimulation, the method including administering a composition comprising a substance that induces CD11b expression in B cells to the subject.

Furthermore, in one aspect, the immunostimulation is preferably stimulation of mucosal immunity. Therefore, the present disclosure provides a method for stimulating mucosal immunity in a subject, the method including administering a composition comprising a substance that induces CD11b expression in B cells to the subject. The stimulation of mucosal immunity includes stimulation of gastrointestinal immunity (for example, intestinal immunity and oral immunity) and nasal immunity, but the stimulation of mucosal immunity is not limited thereto.

Furthermore, in one aspect, the imunostimulation is stimulation of antigen-specific immunity in the mucosa. Therefore, the present disclosure provides a method for stimulating antigen-specific immunity in the mucosa in a subject in need of the stimulation of the antigen-specific immunity in the mucosa, the method including administering a composition comprising a substance that induces CD11b expression in B cells to the subject. The mucosa includes gastrointestinal mucosa (for example, intestinal mucosa and oral mucosa) and nasal mucosa, but the mucosa is not limited thereto.

In one aspect, the immunostimulation includes the effect of maintaining or improving health involving immunostimulation (for example, mucosal immunostimulation and intestinal immunostimulation), the effect of maintaining or improving health involving maintenance or improvement of mucosal flora (for example, intestinal flora), and treatment of an infectious disease (for example, treatment of viral infection, bacterial infection, fungal infection, or parasitic infection), but the immunostimulation is not limited thereto.

Furthermore, in one aspect, the subject in need of the stimulation of antigen-specific immunity in the mucosa is a subject in need of immunostimulation (for example, mucosal immunostimulation and intestinal immunostimulation), a subject in need of maintaining or improving health involving immunostimulation, a subject in need of maintaining or improving mucosal flora (for example, intestinal flora), a subject in need of maintaining or improving health involving mucosal flora, or a subject in need of treatment of an infectious disease (for example, treatment of viral infection, bacterial infection, fungal infection, or parasitic infection).

Therefore, in one aspect, the present disclosure provides a method for stimulating immunity in a subject in need of the immunostimulation (for example, mucosal immunostimulation or intestinal immunostimulation), the method including administering a composition comprising a substance that induces CD11b expression in B cells to the subject.

Furthermore, in one aspect, the present disclosure provides a method for maintaining or improving health improvement through immunostimulation in a subject in need of the maintenance or improvement of health, the method including administering a composition comprising a substance that induces CD11b expression in B cells to the subject.

Furthermore, in one aspect, the present disclosure provides a method for maintaining or improving mucosal flora through immunostimulation in a subject in need of the maintenance or improvement of mucosal flora (for example, intestinal flora), the method including administering a composition comprising a substance that induces CD11b expression in B cells to the subject.

Furthermore, in one aspect, the present disclosure provides a method for treating an infectious disease in a subject in need of the treatment of an infectious disease (for example, treatment of a viral infection, bacterial infection, fungal infection, or parasitic infection), the method including administering a composition comprising a substance that induces CD11b expression in B cells to the subject.

In one aspect, the method for treatment or maintenance or improvement of health of the present disclosure includes a method for inducing migration of activated B cells to a germinal center, the method including preparing a composition comprising a substance that induces CD11b expression in B cells and administering the composition to a subject requiring or requesting the treatment or maintenance or improvement of health.

In one aspect, the method for treatment or maintenance or improvement of health of the present disclosure includes a method for stimulating immunity, the method including preparing a composition comprising a substance that induces CD11b expression in B cells and administering the composition to a subject requiring or requesting the treatment or maintenance or improvement of health. In one aspect, the immunostimulation is preferably stimulation of mucosal immunity and includes stimulation of intestinal immunity.

In one aspect, the method for treatment or maintenance or improvement of health of the present disclosure includes a method for maintaining or improving mucosal flora, the method including preparing a composition comprising a substance that induces CD11b expression in B cells and administering the composition to a subject requiring or requesting the treatment or maintenance or improvement of health. In one aspect, the method for maintaining or improving mucosal flora is preferably a method for maintaining or improving intestinal flora.

In one aspect, the method for treatment or maintenance or improvement of health of the present disclosure includes a method for promoting expression of a high-affinity IgA antibody, the method including preparing a composition comprising a substance that induces CD11b expression in B cells and administering the composition to a subject requiring or requesting the treatment or maintenance or improvement of health.

In one aspect, the method for treatment or maintenance or improvement of health of the present disclosure includes a method for obtaining vaccine adjuvant activity, the method including preparing a composition comprising a substance that induces CD11b expression in B cells and administering the composition to a subject requiring or requesting the treatment or maintenance or improvement of health.

As the substance that induces the CD11b expression in B cells which is used in the method for treatment or maintenance or improvement of health of the present disclosure, any substance described above (the substance that induces the CD11b expression in B cells) can be used.

The composition comprising the substance that induces the CD11b expression in B cells, which is used in the method for treatment or maintenance or improvement of health of the present disclosure, can be prepared by those skilled in the art by a known method. The substance that induces the CD11b expression in B cells can be prepared by mixing the substance with an excipient, a carrier, or the like. Alternatively, the substance can be prepared by obtaining a composition which is obtained by mixing the substance that induces the CD11b expression in B cells with an excipient, a carrier, or the like in advance.

### Examples

Hereinafter, the present invention will be described in more detail with reference to Examples, but these are merely illustrative and do not limit the present disclosure.

### Method

### Mouse Animals

Balb/c mice (8 to 12 weeks old) were obtained from CLEA Japan, Inc. The mice were raised under specific pathogen-free conditions at the Animal Experiment Facility of Institute for Quantitative Biosciences (IQB), The University of Tokyo. All experiments were performed in accordance with the guidelines of Animal Care and Use Committee at the IQB, The University of Tokyo. IgA-Cre/YC3.60^{flox} mice (8 to 12 weeks old) provided by Dr. Takahiro Adachi of Tokyo Medical and Dental University were used for B cell transfer experiments. The IgA-Cre mice were designed based on Reference 38 written by Cris Allen. After mating with the YC3.60^{flox} mice^{39,40}, IgA⁺ cells were identified as YC3.60⁺ cells.

### Flow Cytometry Analysis and Cell Sorting

Peyer's patches (PP) were carefully excised from the small intestines of the Balb/c mice. Single-cell suspensions prepared from the PPs were incubated with the following antibody combinations. Phyacoerythrin-Cy7 (PE-cy7) anti-mouse/human B220 (eBioscience Inc. mAb RA3-6B2), PE anti-mouse/human B220 (BioLegend, Inc. mAb RA3-6B2), FITC anti-mouse/human B220 (BioLegend, Inc. mAb RA3-6B2), PE anti-mouse IgA (α chain-specific) (Southern Biotech), Alexa Fluor (AF) 647 anti-mouse IgA (Southern Biotech), FITC anti-mouse CD11b (BioLegend, Inc. mAb M1/70), and PE anti-mouse CD11b (BioLegend, Inc. mAb M1/70). PE-cy7 anti-mouse CD11b (eBioscience Inc. mAb M1/70), biotinylated PNA (Vector Laboratories, Inc.), and APC-R700 hamster anti-mouse CD95 (Fas) (BD Biosciences mAb Jo2). AF488 anti-mouse CD86 (BioLegend, Inc. mAb GL-1), APC anti-mouse CD184 (CXCR4) (BioLegend, Inc. mAb L236F12), PE anti-mouse IgM (eBiosciences Inc. mAb eB121-15F9), AF647 anti-mouse CD4 (BioLegend, Inc. mAb GK1.5), streptavidin PE (BioLegend, Inc.), streptavidin APC (BioLegend, Inc.), and streptavidin AF488 (Life Technologies Corporation). FSC-H/FSC-W gates were used for single cell selection. Propidium iodide (PI) (NACALAI TESQUE, INC.) was used in order to exclude dead cells. Flow cytometry analysis was performed using Spectral Cell Analyzer SA3800 (Sony Biotechnology Inc.). Cell sorting was performed using Cell Sorter SH800 (Sony Biotechnology Inc.).

### Immunohistochemical Analysis

The separated Peyer's patches were flash frozen in Optimum Cutting Temperature (OCT) Compound (Sakura Finetek Japan Co., Ltd.) and stored at -80°C. PP sections with a thickness of 6 um were prepared and dried overnight. The next day, the PP sections were fixed with acetone (NACALAI TESQUE, INC.), at -20°C for 10 minutes. After washing 5 times with phosphate-buffered saline (PBS), the PP sections were incubated in blocking buffer (PBS/5% FCS (NICHIREI BIOSCIENCES INC.)) for 30 minutes. The PP sections were then incubated with the following antibody combinations in a dark box at RT for 30 minutes or longer. AF488 anti-mouse/human CD11b (BioLegend, Inc. mAb M1/70), PE anti-mouse/human CD11b (BioLegend, Inc. mAb M1/70), AF488 anti-mouse CD4 (BioLegend, Inc. mAb GK1.5), AF647 anti-mouse CD4 (BioLegend, Inc. mAb GK1.5), DAPI solution (BD Bioscience), AF488 anti-mouse CD54 (ICAM-1) (BioLegend, Inc. mAb YN1/1.7.4), AF647 anti-mouse CD54 (ICAM-1 mAb YN1/1.7.4) (BioLegend, Inc.), biotin anti-mouse CD11c (eBioscience Inc. mAb N418), AF488 anti-mouse MAdCAM-1 (BioLegend, Inc. mAb MECA-367), and PE anti-mouse MAdCAM-1 (BioLegend, Inc. mAb MECA-367). Biotinylated PNA, AF647 anti-mouse IgA (α chain-specific) (Southern Biotech), PE anti-mouse IgA (α chain-specific) (Southern Biotech), and streptavidin AF488 (Life Technologies Corporation). Observation was performed using a microscope LSM880 (Carl Zeiss AG). Images were analyzed using ZEN 2009 (Carl Zeiss AG) software.

### Microarray Analysis

RNAs were extracted from 25,000 CD11b⁺IgA⁺ B cells and 100,000 CD11b⁻IgA⁺ B cells using NucleoSpin RNA XS (Takara Bio Inc.). Microarray analysis was performed using SurePrint G3 Mouse GE v2 8x60K Microarray Chip (Affymetrix) to detect gene expression.

### RNA Purification and Real-Time PCR

About 1 × 10⁴ PP GC B cells (PNA^{hi}B220⁺ cells) and about 1 × 10⁴ non-GC PP B cells (PNA^{lo}B220⁺) were each sorted. RNAs were extracted from the isolated cells using NucleoSpin RNA XS (Takara Bio Inc.), and cDNAs were synthesized using GoScript (registered trademark) Reverse Transcriptase (Promega Corporation). Using KAPA SYBR (registered trademark) FAST qPCR Master Mix (2X) Kit (Kapa Biosystems, Inc.), real-time PCR was performed in triplicate on an optical 384-well PCR plate (F. Hoffmann-La Roche Ltd) using LightCycler 480 (F. Hoffmann-La Roche Ltd). Gene expression levels were normalized to the expression level of the housekeeping gene β-actin. The following primers were used for the analysis.
β-actin fw: 5'-CCAACCGTGAAAAGATGACC-3',
β-actin rv: 5'-CCAGAGGCATACAGGGACAG-3',
itgam (CD11b) fw: 5'-CAGATCAACAATGTGACCGTATGGG-3',
itgam (CD11b) rv: 5'-CATCATCATGTCCTTGTACTGCCGCTTG-3',
aicda (AID) fw: 5'-CCTACGCTACATCTCAGACT-3',
AICDA (AID) rv: 5'-CTTTGAAGGTCATGATCCCG-3',
BCL6 (BCL6) fw: 5'-TCCTCACGGTGCCTTTTTACA-3',
bcl6 (BCL6) rv: 5'-TAACGACAAGCATGACGCAG-3',
irf4 (IRF4) fw: 5'-AGGTCTGCTGAAGCCTTGGC-3',
irf4 (IRF4) rv: 5'-CTTCAGGGCTCGTCGTGGTC-3',
s1pr2 (S1PR2) fw: 5'-GTGACGGGACGCAGAGGT-3',
s1pr2 (S1PR2) rv: 5'-AAATGTCGGTGATGTAGGCATG-3',
bcl2 (BCL2) fw: 5'-TGAGTACCTGAACCGGCATCT-3',
bcl2 (BCL2) rv: 5'-GCATCCCAGCCTCCGTTAT-3',
gpr183 (EBI2) fw: 5'-GACATCCTGTTTACCACAGCT-3',
gpr183 (EBI2) rv: 5'-AGACCAGAATCCAGACGGACA-3'

### In vivo Imaging

For imaging of the migration, the IgA-Cre/YC3.60^{flox} mice (8 to 12 weeks old) were anesthetized using a mixture of 3 types of anesthetics, as described in Reference 41. The abdominal walls were carefully incised to expose the small intestines. PPs were visually identified. About 8,000 isolated CD11b⁺IgA⁺ PP B cells and about 30,000 CD11b⁻IgA⁺ PP B cells were labeled with the CellTracker Orange CMTMR fluorescent dye (Invitrogen Corporation) and then directly injected into the PPs of IgA-Cre/YC3.60^{flox} transgenic mice using a 25-µL syringe (Trajan Scientific and Medical). The PPs into which the cells were transplanted were observed using an LSM880 microscope (Carl Zeiss AG). Images were analyzed using the ZEN 2009 software (Carl Zeiss AG). After observing the PPs using the microscope for 1 hour, the abdominal incision sites were carefully closed using an ELP skin stapler (Akiyamaseisakusyo. Co., Ltd.). 40 hours after the transplant, the PPs containing the transplanted cells were observed again using the LSM880 microscope (Carl Zeiss AG) under anesthesia, whereby the localization of the transplanted CD11b⁺IgA⁺ PP B cells was confirmed, and analysis was performed using the ZEN 2009 software (Carl Zeiss AG).

### Conjugation Analysis

Conjugation between the CD11b⁺IgA⁺ PP B cells and CD4⁺ PP T cells was analyzed by flow cytometry using Cell Sorter SH800 (Sony Biotechnology Inc.). Singlet cells were selected using FSCH/FSC-W gates. The singlet cells were selected using the FSCH/FSC-W gates, and the conjugated cells were negatively selected by singlet cell gate discrimination. The conjugated cells were isolated from the PP conjugated CD11b⁺IgA⁺CD4⁺ for imaging, and observation was performed using the microscope LSM880 (Carl Zeiss AG) and the ZEN 2009 (Carl Zeiss AG) analysis software.

### Isolation of CD11b⁺IgA⁺ PP B Cells Using iGB Culture System

About 800 sorted CD11b⁺IgA⁺ PP B cells and 800 sorted CD11b⁻IgA⁺ PP B cells were dyed with CellTrace Violet Proliferation Kit (Invitrogen Corporation). Since the number of isolated CD11b⁺IgA⁺ PP B cells was very low, 5,000 CD11b⁻IgA⁺ PP B cells and 5 × 10⁴ naive splenic B cells (negatively isolated by B cell isolation kit (Miltenyi Biotec B.V. & Co. KG)) were prepared as positive controls. To monitor cell proliferation, the sorted CD11b⁻IgA⁺ PP B cells, CD11b⁺IgA⁺ PP B cells and naive splenic B cells were seeded in a 6-well tissue culture dish in the presence of 40LB cells pretreated with mitomycin C to inhibit growth. The cells were cultured at 37°C and 5% CO2 for 3 days, using RPMI-1640 medium (Wako Pure Chemical Industries, Ltd.) (containing 10% FCS, 5.5 × 10⁻⁵ M 2-mercaptoethanol (ME) (NACALAI TESQUE, INC.), and 10 mM HEPES (NACALAI TESQUE, INC.)). The isolated CD11b⁺IgA⁺ PP B cells and CD11b⁻IgA⁺ PP B cells were cultured with rIL-21 (10 ng/ml; PeproTech, Inc.), and the naive splenic B cells were cultured with rIL-4 (1 ng/ml; BioLegend, Inc.). Flow cytometry analysis was performed using an iGB culture system to detect CellTrace Violet from Day 0 to Day 3. The CD11b expression in the cultured cells was analyzed by flow cytometry on Day 0 and Day 1 using Cell Sorter SH800 (Sony Biotechnology Inc.) and Spectral Cell Analyzer SA3800 (Sony Biotechnology Inc.).

### Splenic CD11b⁺B Cells Stimulated with Pam3CSK4 on iGB Culture System

As for culturing splenic CD11b⁺ B cells in the iGB culture system, splenic naive B cells were cultured with pam3CSK4 (InvivoGen) for 3 days, and the expression of CD11b was induced. Next, 1.5 × 10⁵ CD11b⁺ splenic B cells and 1.5 × 10⁵ CD11b- splenic B cells were isolated by flow cytometry, dyed with CellTrace Violet (Invitrogen Corporation), and then cultured for 4 days using IL-4 (1 ng/ml; BioLegend, Inc.) in a 40LB system. Next, using Cell Sorter SH800 (Sony Biotechnology Inc.) and Spectral Cell Analyzer SA3800 (Sony Biotechnology Inc.), the CD11b expression was analyzed by flow cytometry.

### Preparation of Bacteria for Stimulating B Cells

E. coli (DH5α) and S. Enterica were seeded in 5 ml of LB medium from a glycerol stock and cultured by shaking at 180 rpm overnight at 37°C. On Day 2, 0.5 ml of the cell suspension was re-suspended in 10 ml of LB medium. The culture solution was subjected to shake culture at 37°C for 3 hours, and the growth curve of the culture solution was observed using the optical density (OD) at a wavelength of 600 nm (OD600) detected every 30 minutes. 100 µl of serial dilution solution of the cell suspension was dispersed on an LB plate, and CFU was calculated. Based on the growth curve, the E. coli and S. Enterica were cultured and harvested, and heat-killed in an autoclave (121°C, 15 minutes).

Similarly, B. bifidum and B. breve were anaerobically cultured in a Difico Lactobacilli MRS broth (Becton, Dickinson and Company) medium at 37°C, and heat-killed in an autoclave.

### Stimulation of Splenic B cells (in vitro)

Splenic B cells were negatively sorted from the spleens of 8-week-old Balb/c mice using a B cell isolation kit (Miltenyi Biotec B.V. & Co. KG). The splenic B cells (concentration: 5 × 10⁵/ml) were cultured in RPMI1640 medium, and 13 types of independent stimuli were each applied thereto. The results of titrating each stimulus to select the appropriate concentration of each stimulus are shown in Figs. 11e to g.

1. Anti-mouse IgM (15 ug/ml; Jackson ImmunoResearch Laboratories Inc.)
2. Anti-mouse-CD40 (HM40-3, 500 ng/ml; manufactured by BioLegend, Inc.)
3. BAFF (50 ng/ml; BioLegend, Inc.)
4. Pam3CSK4 (1 ug/ml, InvivoGen)
5. Poly I:C (25 ng/ml, InvivoGen)
6. LPS (50 ng/ml, Sigma-Aldrich, Inc.)
7. Flagellin (20 ng/ml, Sigma-Aldrich, Inc.)
8. Imiquimod (R837, 5 ng/ml; InvivoGen)
9. CpG (20 ug/ml, GeneDsign Inc.)
10. Heat-killed E. coli (10⁷ CFU/ml)
11. Heat-killed S. Enterica (10⁸ CFU/ml)
12. Heat-killed B. bifidum (10⁷ CFU/ml)
13. Heat-killed B. breve (10⁷ CFU/ml)

The CD11b expression in the stimulated B cells was analyzed on Day 0 and Day 3 using Cell Sorter SH800 (Sony

Biotechnology Inc.) and Spectral Cell Analyzer SA3800 (Sony Biotechnology Inc.).

For MDP stimulation, purified splenic B cells (5 × 10⁵/ml) were cultured in RPMI1640 medium supplemented with N-acetylmuramyl-L-alanyl-D-isoglutamine hydrate (1 ug/ml; Sigma-Aldrich Inc.).

To analyze a TLR inhibitor and a NOD2 inhibitor, purified splenic B cells (5 × 10⁵/ml) were cultured in RPMI1640 medium containing a TLR1/2 antagonist CU-CPT (1 µM; Sigma-Aldrich Inc.), a TLR4 inhibitor TAK-242 (1 µM; Sigma-Aldrich Inc.), or NOD2 signaling inhibitor II, and GSK717 (30 µM; Sigma-Aldrich Inc.) in the presence of pam3CSK4 (1 µg/ml, InvivoGen), LPS (50 ng/ml; Sigma-Aldrich Inc.), CpG (20 µg/ml, GeneDsign Inc), and heat-killed E. coli (10⁷ CFU/ml).

### Immunization

On Day 0 and Day 16, the mice were orally administered with OVA (1 mg), OVA (1 mg) + pam3CSK4 (10 µg, InvivoGen), or OVA (1 mg) + heat-killed E. coli (10⁹ CFU in 100 µl). Stools were collected on Day 21, and OVA-specific antibody measurement was performed by enzyme-linked immunosorbent assay (ELISA).

### ELISA Detection of OVA-Specific IgA

The stools were suspended in 10-times weight/volume (w/v) PBS. After performing centrifugation (8,000 g, 15 minutes), the supernatant was collected as a fecal extract. A plate was precoated with 1 mg/ml OVA overnight, followed by blocking with PBS containing 1% (w/v) bovine serum albumin at room temperature for 1 hour. Then, a serially diluted fecal extract was added to the plate, and the plate was incubated at room temperature for 1 hour. Relative avidity of IgA was detected using alkaline phosphatase-labeled goat anti-mouse IgA (manufactured by Southern Biotech). After incubation overnight at 4°C, optical density (OD) values at 405 nm were measured using TriStar Multimode Reader LB 942 (manufactured by Berthold Technologies GmbH & Co.KG).

### Oral Administration of Heat-Killed Bacteria to WT Mice

Balb/c mice (8 to 12 weeks old) were orally administered with heat-killed E. coli (10⁹ CFU diluted in 100 ul of PBS), S. Enterica (10⁹ CFU diluted in 100 ul of PBS), B. bifidum (10⁹ CFU diluted in 100 ul of PBS), B. breve (10⁹ CFU diluted in 100 ul of PBS), or pam3CSK4 (10 µg diluted in 100 µl of PBS). Mice orally administered with 100 ul of PBS were prepared as controls. 2 days later, the PPs of these mice were excised, and B cells in the GCs were analyzed using Spectral Cell Analyzer SA3800 (Sony Biotechnology Inc.), as described above.

### Administration of Oral Adjuvant using OVA as Antigen

Using OVA 1 mg as an antigen, each adjuvant (CpG-K3 (GeneDsign Inc.) 10 µg, CpG-ODN1826 (Invirogen Corporation tlrl-1826) 10 µg, or heat-killed E. coli 10⁹ c.f.u.) was mixed with the antigen, C57BL/6 mice were subjected to oral immunization 3 times every 2 weeks, and blood was collected on the second day after the third immunization. Serum TNF-α levels were measured in accordance with the protocol of mouse TNF-α ELISA kit (Invitrogen Corporation, BMS607-3).

### In Vivo Imaging of Transplanted iGB cells Induced by Pre-Stimulation

As described above, splenic naive B cells were cultured separately in vitro for 3 days using pam3CSK4 (1 ug/ml, InvivoGen), heat-killed E. coli (10⁷ CFU/ml), CpG (20 ug/ml, GeneDsign Inc), or anti-IgM-Ab (15 µg/ml, Jackson ImmunoResearch Laboratories Inc.). Then, 1 × 10⁵ B220⁺ B cells were isolated from the stimulated cells, and subsequently, the cells were seeded onto 40LB feeder cells supplemented with rIL-4 (1 ng/ml; BioLegend, Inc.) to induce iGB cells. As a control group, splenic naive B cells not subjected to stimulation were seeded onto the 40LB feeder cells supplemented with rIL-4 (1 ng/ml; BioLegend, Inc.). After culturing for 4 days, naive B-40LB cells, pam3CSK4-40LB cells, E. coli-40LB, IgM-40LB, and CpG-40LB cells of 3 × 10⁵ iGB cells (B220⁺) were isolated, labeled with CellTracker Orange CMTMR fluorescent dye (Invitrogen Corporation), and then separately injected intravenously into mice (Balb/c, 8 to 12 weeks old) together with 10 µg of AF488 anti-mouse MAdCAM-1 (BioLegend, Inc. mAb MECA-367) to identify HEV. Under anesthesia, 1 µg of AF647 anti-mouse IgA (Southern Biotech) was directly injected into the PPs of the same mice into which iGB cells were transferred, to stain the IgA⁺ cells for GC identification. Localization of intravenously injected labeled iGB cells was observed using a microscope LSM880 (Carl Zeiss AG) and ZEN 2009 (Carl Zeiss AG) analysis software.

### Amplification of DNA in Rearranged VDJ Region and Downstream Intron

The rearranged VDJ sequence with a downstream intronic sequence was amplified by 2 rounds of nested PCR using several different upstream primers (here, "S" is C or G, "R" is A or G, "N" is A, G, C, or T, "M" is A or C, and "W" is A or T). MH1, 5'SARGTNMAGCTGSAGSAGTC-3'; MH2, 5'-SARGTNMAGCTGSAGSAGTCWGG-3'; MH3, 5'-CAGGTTACTCTGAAAGWGTSTG-3'. MH4, 5'--GAGGTCCARCTGCAACARTC-3'; MH5, 5'-CAGGTCCAACTVCAGCARCC-3'; MH6, 5'-GAGGTGAASSTGGTGGAATC-3'. MH7,5'-GATGTGAACTTGGAAGTGTC-3' was used together with the JH4R primer (5'-GACTAGTCCTCTCCAGTTTCGGCTGAATCC-3')^{16,42} which is complementary to the sequence located at the 5' end of the IgH intron enhancer. In the second PCR, the same upstream primer was used together with the JH4R-2 primer (5'-CAGGTGGTGTTTTGCTCA-3') which is complementary to the upstream sequence of the JH4R primer. In the amplification, PrimeSTAR Max DNA Polymerase (manufactured by Takara Bio Inc.) was used, and 20 cycles of first round PCR and 30 cycles of second round PCR (95°C 15 seconds, 58°C 10 seconds, and 72°C 30 seconds) were performed. After cloning the PCR product into pGEM T vector (Promega Corporation), colony PCR was performed with the JH4F primer (5'-TAT GCT ATG GAC TAC TGG-3') and the JH4R-2 primer using EmeraldAmp (registered trademark) PCR Master Mix (Takara Bio Inc.), and clones containing the intronic sequence were selected.

### DNA Sequence and SHM Analysis

After separating the plasmid DNA, DNA sequence analysis was performed using the T7 primer and the SP6 primer. The consensus of the IgH intronic DNA sequence was obtained from Accession No. AJ 851868.3. The rearranged VDJ was analyzed by IgBLAST.

### Statistical Analysis

Unless otherwise stated, statistical analysis was performed using GraphPad Prism version 8.4.2 for Mac (GraphPad Software, San Diego, CA, USA). Differences between 2 groups were compared using a two-tailed Student's t test. In a case where there are 3 or more groups, one-way or two-way analysis of variance (ANOVA) was performed, followed by Tukey's multiple comparison test. Details regarding statistical tests and number of groups and replications are provided in the legend in the drawing. A p value < 0.05 was considered significant.

### Example 1: Small Population of IgA⁺ B Cells Expressing CD11b in Mouse PP

The presence of a small population of CD11b⁺IgA⁺ B cells (about 0.1% to 0.2% of PP B cells) in PP was confirmed by flow cytometry (Figs. 2a and 6a). Both CD11b⁺IgA⁺ and CD11b⁻IgA⁺ PP B cells exhibited typical GC B cell phenotypes such as Peanut Agglutinin (PNA)^{high} and Fas⁺¹⁶ (Figs. 2b and c) .

Next, since pre-GC B cells exist in the IF region (outside GC) before entering the GC, the localization of CD11b⁺IgA⁺ B cells was investigated^{3,4}. Immunohistologically, the GC region was specified by expression of both PNA and IgA (Fig. 2d). As a result, it was found that only two CD11b⁺IgA⁺ B cells (yellow cells) were present in the region outside the sectioned GC (Figs. 2d and 7). Next, the localization of CD11b ligand was analyzed. Intercellular adhesion molecule 1 (ICAM-1) is widely known as a ligand for CD11b/CD18 that mediates cell adhesion and migration¹⁷. As previously reported^{8,18}, both GC B cells and high endothelial venules (HEV) in the IF region express ICAM-1. Furthermore, HEV also expresses mucosal addressin cell adhesion molecule-1 (MAdCAM-1)¹⁹ (Figs. 2e and 8). Although direct binding of CD11b⁺IgA⁺ PP B cells to HEV could not be confirmed, in the IF region of the PP section, 6 out of 7 CD11b⁺IgA⁺ PP B cells were found to be located near HEV (Fig. 2f). Another CD11b⁺IgA⁺ PP B cell (No.7) was located near GC (Fig. 2f), but was not present within the GC, suggesting that the cell may have migrated from the IF to the GC.

The next question was whether the CD11b⁺IgA⁺ PP B cells subsequently migrated from outside the GC into the GC. CD11b⁺IgA⁺ and CD11b⁻IgA⁺PP B cells were isolated, labeled, and separately injected into the PPs of different IgA-Cre/YC3.60^{flox} reporter mice. Since the GC of PP can be identified in a region enriched with IgA⁺ B cells (Fig. 2d), in the reporter mice which fluoresce YC3.60 in IgA⁺ cells, GCs in the PPs can be easily identified. 1 hour after the injection, the injected CD11b⁻IgA⁺ B cells were located inside the GC (Fig. 2g), suggesting that the CD11b⁻IgA⁺ B cells were GC B cells. Meanwhile, the injected CD11b⁺IgA⁺ B cells surrounded the GC, instead of entering the GC (Fig. 2g). 40 hours after the injection, the injected CD11b⁺IgA⁺ B cells also entered the GC (Fig. 2h). In this manner, the CD11b⁺IgA⁺ B cells which were originally in the IF region migrate to the GC.

### Example 2: Gene Expression in CD11b⁺IgA⁺ PP B Cells

To further confirm the phenotype of pre-GC B cells, CD11b⁺IgA⁺ and CD11b⁻IgA⁺ PP B cells were isolated and the gene expression was compared by microarray analysis (Figs. 3a and 6b). It was shown that CD11b⁺IgA⁺ B cells expressed GC genes at a relatively low level, and non-GC genes at a relatively high level, compared to the CD11b⁻IgA⁺ B cells, and the CD11b⁺IgA⁺ B cells were a different population from the CD11b⁻IgA⁺ B cells. To confirm the microarray results, quantitative PCR (qPCR) was further performed (Figs. 3b and 6b) to compare the gene expression in the CD11b⁺IgA⁺ B cells and the gene expression in GC (PNA^{high}B220⁺) and non-GC (PNA^{low}B220⁺) PP B cells. The inventors selected 3 genes that are highly expressed in GC B cells, including bcl6 (a key transcription factor restricted in GC B cells), aicda (an enzyme that encodes activation-induced cytidine deaminase (AID) and induces somatic hypermutation (SHM) and class switch recombination), and s1pr2 (a receptor that controls the position of B cells within and outside GC)^{10,20-22}, and 2 non-GC genes, gpr183 (a receptor that controls the position of B cells outside a follicle) and bcl2 (a transcription factor highly expressed in non-GC B cells)^{10,23,24}. The expression levels of these genes in CD11b+IgA+ and CD11b⁻IgA⁺ PP B cells are different from those in non-GC B cells (Fig. 3b), indicating that both of these two populations are likely to be GC B cells. Since the expression level of the transcription factor irf4, which has been demonstrated to be required for initiation of GC response, is increased in pre-GC B cells^{10,11,25}, the expression of irf4 was further analyzed. The irf4 expression was higher in CD11b⁺IgA⁺ PP B cells than in CD11b⁻IgA⁺ PP B cells (Fig 3b), thus finding that the CD11b⁺IgA⁺ PP B cells were the pre-GC B cells. However, taking into account that some of the B cells in the GC light zone (LZ) also highly express irf4^{8,25}, the expression of CXCR4 and CD86 in CD11b⁺IgA⁺ PP B cells was subsequently analyzed by flow cytometry. Since GC LZ B cells are identified as CD86^{high}CXCR4^{low} B cells^{3,8}, compared to CD11b⁻IgA⁺ B cells,
the CD86 expression is low, and the CXCR4 expression is high in CD11b⁺IgA⁺ B cells (Figs. 3c and 6c), thus indicating that the CD11b⁺IgA⁺ B cells were pre-GC B cells, rather than GC LZ B cells.

### Example 3: CD11b⁺IgA⁺ PP B Cells Interact with T Cells Before Entering GC

To enter GC, pre-GC B cells need to interact with T cells and DCs, as previously reported^{3,26}. Therefore, whether CD11b⁺IgA⁺ PP B cells interact with CD4⁺ T cells before entering GC was investigated. IgA⁺ PP B cells were not CD4⁺ positive by single B cell gate by flow cytometry (Figs 3d and 9). However, a distinct population of IgA⁺CD4⁺ double-positive PP cells was present in conjugate B cells (Figs. 3d and 9), indicating that these IgA⁺ PP B cells interact with CD4⁺ T cells. Particularly, 40% or more of the CD11b⁺IgA⁺ B cells were shown to be bound to CD4⁺ T cells (Figs. 3e and f and 9), suggesting that CD11b⁺IgA⁺ PP B cells interact with CD4⁺ T cells. On the other hand, about 14.5% of CD11b⁻IgA⁺ PP B cells interact with CD4⁺ T cells, and there is a possibility that, in GC LZ, the CD11b⁻IgA⁺ PP B cells interact with CD4⁺ T follicular helper (T_{FH}) cells (Figs. 3e and f)^{4,8}. When conjugated cells separated from PP are photographed with a confocal microscope, the interaction between CD11b⁺IgA⁺ PP B cells and CD4⁺ T cells was further confirmed (Fig. 3g). Thus, the present results clearly indicate that CD11b⁺IgA⁺ PP B cells are pre-GC PP B cells.

### Example 4: CD11b⁺IgA⁺ PP B Cells Accumulate SHMs in Ig Gene

To confirm whether CD11b⁺IgA⁺ B cells are derived from newly activated naive B cells, the sequences of both the rearranged VDJ region and the downstream intronic sequence were determined in IgH of CD11b⁺IgA⁺ and CD11b⁻IgA⁺ PP B cells, revealing that SHM was introduced during the GC response (Figs. 10a and b)¹⁶. Mutations were found in 80% or more of the sequences in CD11b⁺IgA⁺ PP B cells (21 out of 25 clones) and CD11b⁻IgA⁺ PP B cells (32 out of 39 clones) (Fig. 10b). This indicates that CD11b⁺IgA⁺ PP B cells are derived not only from newly activated naive B cells but also from memory B cells that have re-entered GC, as previously reported^{6,7}.

### Example 5: Bacterial Antigens Induce CD11b Expression in Naive Splenic B Cells

The findings of the inventors on CD11b⁺IgA⁺ PP B cells raise another question: what causes the CD11b expression in pre-GC B cells. B cells transform into pre-GC B cells not only by stimulation with an antigen but also by interaction with DCs and T cells^{3,4,26}. To investigate what kind of signal induces the CD11b expression, the inventors isolated naive splenic B cells from non-immunized mice and cultured the cells in vitro using anti-IgM to cross-link BCR, B-cell activating factor (BAFF) to interact with DCs, and anti-CD40 to interact with T cells. However, these stimuli that mimicked the T-B and DC-B interactions did not induce CD11b in naive B cells. Next, naive B cells were stimulated using different Toll-like receptor (TLR) ligands, such as pam3CSK4 for TLR2, poly I:C for TLR3, lipopolysaccharide (LPS) for TLR4, flagellin for TLR5, imiquimod for TLR7, and CpG for TLR9. After 3 days of culturing, the number of B cells stimulated by pam3CSK4, LPS, and CpG was significantly increased compared to the B cells stimulated with anti-IgM, anti-CD40, BAFF, poly I:C, flagellin, and imiquimod (Figs. 11a and b). The proportions of CD11b⁺ B cells in the B cells stimulated with pam3CSK4 and LPS were 27.6% and 8%, respectively, whereas the proportions of CD11b⁺ B cells in the B cells stimulated with other stimuli were 5% or less (Figs.4-1a and b). What is notable is that while B cell proliferation was activated by CpG, CD11b expression was not induced by CpG. Furthermore, expression of itgam (CD11b) in B cells was confirmed to be increased only in the case of stimulating with pam3CSK4 and LPS, which are known to be bacterial antigens (Fig 4-1c). Next, when heat-killed E. coli was prepared that strongly stimulates both TLR2 and (or) TLR4^{27,28}, the heat-killed E. coli significantly induced CD11b expression in splenic B cells (Fig. 4-1a). From these results, it was found that bacterial antigens which are ligands for TLR2 and TLR4 induce CD11b in B cells.

Since B cells inside PP GCs do not express CD11b (Fig. 2d), it is considered that the CD11b expression in B cells is transient and converges through the T-B and DC-B interactions. Thus, using 40LB cells expressing CD40L and BAFF²⁶ as a feeder layer, the surface phenotype was tracked by using in vitro induced GC B cell (iGB) culture system that mimics the roles of T cells and DC cells and induces GC-like B cells. When cells are cultured in the iGB system, isolated CD11b⁺IgA⁺ PP B cells were able to proliferate at levels similar to those of isolated CD11b⁻IgA⁺ PP B cells and naive splenic B cells (Figs. 12a and b). In fact, the isolated CD11b⁺IgA⁺ PP B cells lost the CD11b expression when cultured in the iGB system for 1 day (Figs. 4-1d and 12a). Similarly, when CD11b⁺ splenic B cells induced by pam3CSK4 were cultured in the iGB system for 4 days, the CD11b expression was lost (Figs. 4-1e and 12c). In splenic naive B cells treated with anti-CD40 Ab and anti-IgM Ab, the CD11b expression was significantly suppressed even in the presence of pam3CSK4 (Figs. 4-1f to h). This indicates that the interaction between T-B cells and BCR cross-linking downregulate the CD11b expression in B cells. Therefore, it is reasonable that only a small number of CD11b⁺IgA⁺ PP B cells are found in the living body (Fig. 2).

To further confirm whether TLR2 and TLR4 are involved in the induction of CD11b expression in splenic B cells, splenic B cells were stimulated with pam3CSK4, LPS, CpG, and heat-killed E. coli in the presence of a TLR1/2 inhibitor, a TLR4 inhibitor, and a NOD2 inhibitor. NOD2 is known to be involved in signaling downstream of TLR2²⁹. Interestingly, pam3CSK4 and heat-killed E. coli increased the proportion of CD11b⁺ B cells when the TLR1/2 inhibitor was used, whereas the proportion did not increase with the TLR4 inhibitor (Fig. 4-1i). Meanwhile, muramyl dipeptide (MDP), a ligand for NOD2, did not induce the CD11b expression, but the NOD2 inhibitor strongly suppressed the CD11b expression in B cells stimulated with pam3CSK4 and heat-killed E. coli (Fig. 11d). Such result strongly suggests that the CD11b expression is partially induced by bacteria-derived stimulation via NOD2, instead of being directly induced by the activation of TLR2 and TLR4 (Fig. 4-1i).

When heat-killed Klebsiella pneumoniae belonging to the phylum Proteobacteria and Bacteroides vulgatus belonging to the phylum Bacteroidetes were tested in the same manner as E. coli, it was confirmed that these bacteria also induce the CD11b expression. The proportions of CD11b⁺ B cells in B cells after stimulation with each of the bacteria are shown in Fig. 4-2. The present result indicates that bacteria belonging to the phylum Proteobacteria and bacteria belonging to the phylum Bacteroidetes induce the CD11b expression in naive splenic B cells.

### Example 6: Promotion of GC Response by Oral Administration of Heat-Treated CD11b-Inducing Bacteria

Next, the question was what kind of bacteria can induce pre-GC B cells through CD11b expression. E. coli and S. Enterica were selected as harmful bacteria, and Bifidobacterium bifidum (B. bifidum) and B. breve were selected as representative beneficial bacteria. E. coli and S. Enterica induced the CD11b expression by in vitro stimulation, while B. bifidum and B. breve did not induce the CD11b expression (Figs. 5a and b). When pam3CSK4 and heat-killed bacteria were orally administered to mice, the number and proportion of PP GC B cells increased within 2 days (Figs. 5c to e). This suggested that only harmful bacteria can enhance the ongoing GC response in the living body.

### Example 7: Transient Expression of CD11b Allows cultured B cells to enter GC.

Next, whether CD11b⁺ B cells induced in vitro can enter existing GC was determined. To induce GC B cells in vitro, CD11b was induced by stimulating splenic naive B cells with pam3CSK4 or heat-killed E. coli in vitro, and then the cells were cultured on 40LB cells for 4 days. When iGB cells stimulated in advance with pam3CSK4 and heat-treated E. coli, which are the stimuli that induces CD11b, were intravenously injected into mice, most of the cells entered GC, whereas iGB cells not expressing CD11b after stimulation with CpG and anti-IgM in advance did not enter GC (Figs. 5f and 13). The above results indicate that expressing CD11b in B cells before the T-B or DC-B interaction is essential for pre-GC B cells to enter GC. This is consistent with previous report that a bacterial antigen, which is a non-self antigen, is important for triggering GC responses³⁰.

### Example 8: CD11b-Inducing Heat-Killed Bacteria Act as Effective Mucosal Adjuvant and Induce Antigen-Specific Mucosal IgA

Adjuvants based on TLR2 or TLR9 ligand have already been widely used for vaccination, since the ligand effectively stimulates DCs^{31,32}. However, the results of the inventors indicate that B cells can decide on their cell fate to become pre-GC B cells before receiving help from DCs or T cells. Therefore, the inventors thought that pam3CSK4 and heat-treated E. coli may function as an adjuvant for a mucosal vaccine. The inventors orally administered ovalbumin (OVA) to mice as an antigen, and, in addition, orally administered pam3CSK4 or heat-killed E. coli as an adjuvant (Fig. 5g). As expected, OVA-specific IgA increased in the stools of mice simultaneously administered with OVA and heat-killed E. coli or pam3CSK4, while OVA-specific IgA did not increase in mice only administered with OVA (Fig. 5h). Thus, in this study, it was revealed that CD11b, a pre-GC surface marker on B cells, is a promising marker for selecting an effective mucosal vaccine adjuvant, and particularly, CD11b enhances GC-derived high-affinity mucosal IgA.

Meanwhile, as a result of measuring the cytokine concentration after the third immunization, the adjuvant composition of the present disclosure did not increase the serum TNFα concentration at all. On the other hand, CpG-K3 and CpG-ODN1826, known vaccine adjuvants, were thought to cause increase in the serum TNFα concentration and induce an inflammatory response (Fig.14).

These results confirmed that the adjuvant composition of the present disclosure has extremely beneficial properties in that it has a high ability to induce antigen-specific binding IgA without inducing an inflammatory response.

### (Discussion)

In the present study, by investigating different populations of CD11b⁺IgA⁺ B cells, it was shown that CD11b is a new surface marker for pre-GC IgA⁺ B cells. Induction of CD11b in B cells depends on the stimulation with harmful bacterial antigens, but is independent of activated DCs. When these CD11b-inducing bacterial antigens were orally administered to mice, a mucosal antigen-specific IgA response was enhanced in vivo. These results indicate that allowing transient expression of CD11b in activated B cells before entering GC is an important step for selecting activated B cells suitable for GC response.

Regarding the function of CD11b in B cells, Yan's group revealed that CD11b is important for regulating the BCR signaling through the Lyn-CD22-SHP-1 negative feedback pathway¹³. In CD11b-/- mice, antibody production was enhanced, and a GC response by autoreactive B cells was observed^{13,14}. In addition, mutations in the ITGAM gene which encodes CD11b have been reported to be a high-risk factor for developing autoimmune diseases such as systemic lupus erythematosus (SLE)³³. In the present disclosure, the CD11b expression was confirmed to be a marker for B cells before entering GC. Presumably, it is considered that the transient expression of CD11b is not necessary but sufficient for entry into GC. When B cells are activated by stimulation with bacteria, the B cells express CD11b and prevents their proliferation and production of self-reactive antibodies. Then, the B cells start to interact with T cells in the IF region. After the T-B interaction, activated B cells lose the CD11b expression, enter GC DZ, rapidly proliferate, and undergo SHM to obtain high affinity. It is considered that CD11b plays an important role in controlling activated B cells to cause beneficial GC responses, but also controls to avoid harmful self-reactions.

Do human B cells also express CD11b in response to microbial antigens? Dutra's group has demonstrated that stimulation of B cells from Chagas' disease patients with Trypanosoma cruzi-derived protein-enrich fraction increases the frequency of CD11b⁺B cells³⁴.

Moreover, it was clarified what kind of signal induces the CD11b expression. First, a TLR2 ligand pam3CSK4 and heat-killed E. coli strongly induced CD11b in B cells in vitro, and thus it was predicted that TLR2 stimulation induces CD11b. E. coli has peptidoglycan (PGN) which is a component of the cell wall, and the peptidoglycan serves as a ligand for TLR2³⁵. However, unexpectedly, the combination of pam3CSK4 and TLR1/2 inhibitors promoted CD11b expression in B cells. On the other hand, the CD11b expression induced by pam3CSK4 and heat-killed E. coli was suppressed by a NOD2 inhibitor (Fig. 4-1i), and thus it was suggested that NOD2 regulates CD11b. Such a result is partially consistent with previous findings that NOD2 acts as a negative regulator of TLR2³⁵. In addition, NOD2 contributes to the maintenance of immune tolerance and homeostasis by suppressing the induction of TLR2/4-mediated inflammatory cytokine production, which is strongly associated with Crohn's disease (CD)³⁶. This suggests that the TLR2-NOD2 pathway may not only control inflammatory responses but also GC responses through CD11b. However, other studies have shown that the TLR1/2 signaling pathway is independent of NOD2³⁷. It is expected that further research will clarify the details, including the fact that CD11b induction is promoted by a TLR2 inhibitor.

As for CD11b-inducing bacterial stimulation, S. Enterica and E. coli, which are examples of harmful bacteria, were shown to induce CD11b expression in vitro, and enhance GC responses in vivo. Klebsiella pneumoniae and Bacteroides vulgatus, bacteria of the class Bacteroidia, but not the genus Bifidobacterium, were shown to induce the CD11b expression in vitro.

In summary, the following was clarified from the experiments of the present disclosure.
1. CD11b⁺IgA⁺ B cells express PNA and FAS which are surface markers for GC B cells. CD11b⁺IgA⁺ B cells highly express GC B-specific genes, but do not express non-GC genes.
2. CD11b⁺IgA⁺ B cells highly express irf4 which is a pre-GC gene.
3. CD11b⁺IgA⁺ B cells are located outside GC and are about to enter GC.
4. CD11b⁺IgA⁺ B cells interact with CD4⁺ T cells outside GC.
5. In vitro stimulation with a bacterial antigen induces CD11b expression in splenic B cells.
6. An orally administered bacterial antigen promotes GC response within 2 days in the living body.
7. Using heat-treated E. coli as an adjuvant of an oral vaccine can enhance an antigen-specific response by inducing pre-GC B cells.

That is, the present disclosure clarifies the following.
(1) CD11b⁺IgA⁺ B cells are pre-GC B cells
(2) Induction of CD11b in B cells is a promising marker for selecting an effective mucosal immunization vaccine adjuvant.

It is also suggested that B cells can sense a bacterial antigen and determine their own cell fate into pre-GC cells, independent of DCs and T cells (Fig. 15).

Mucosal immunity-related immunity-inducing tissues undergo affinity maturation of IgA+ B cells that recognize antigen molecules taken in from mucosal tissue epithelium or the like, and secrete a high-affinity antibody from mucosal epithelium, thereby having an important function of providing antigen-specific defense to the mucosal tissues. Mucosal immunity-related immunity-inducing tissues exist in various mucosa such as intestinal mucosa (Peyer's patch) and nasal mucosa, and the immune cells activated in such mucosa provide antigen-specific defense in the tissues of the mucosa or a distal mucosa (for example, The Japanese journal of Oto-Rhino-Laryngology 114: 843-850, 2011, Vaccine Vol. 30, pp. 180-188, 2012, and Frontiers in Immunology, 2021, Vol. 12, Article 635471). According to each Example of the present disclosure, Peyer's patch germinal center was used as a model for the mucosal immunity-related immunity-inducing tissue, and it became clear that CD11b⁺IgA⁺ B cells enter the germinal center (GC) and undergo affinity maturation to produce a high-affinity antibody. It also became clear that heat-sterilized bacteria, which induces the CD11b expression in IgA⁺ B cells, can be used as an extremely useful mucosal immunization vaccine adjuvant that suppresses the induction of inflammatory responses.

The present disclosure indicates that an adjuvant that induces CD11b⁺IgA⁺ B cells is extremely useful in affinity maturation of B cells in germinal centers of various mucosal immunity-related immunity-inducing tissues and induction of high-affinity IgA antibody production, and heat-sterilized bacteria can be used as the adjuvant.

Mucosally secreted IgA antibodies are known to exhibit polyreactive reactivity against bacteria, and can provide antigen-specific immune defense against a wider range of antigens than IgG antibodies, which are induced to be produced by general vaccines and the like (for example, Journal of Intestinal Microbiology 31: 151-157, 2017, Science. 2017 Oct 20; 358 (6361): eaan6619., and Cell Reports 36, 109655, 2021). In one aspect, the adjuvant of the present disclosure is extremely useful in that it can provide polyreactive antigen-specific immune defense while suppressing inflammatory responses, and will bring about significant contribution in the fields of medicine, food, feed, and the like.

### (References)

1. Turner, J. S. et al. Human germinal centres engage memory and naive B cells after influenza vaccination. Nature. 586, 127-132 (2020).
2. Andersen, T. K. et al. Enhanced germinal center reaction by targeting vaccine antigen to major histocompatibility complex class II molecules. npj Vaccines. 4, 9 (2019).
3. Mesin, L., Ersching, J., & Victora, G. D. Germinal center B cell dynamics. Immunity. 45.3, 471-482 (2016).
4. Kurosaki, T., Kometani, K., & Ise, W. Memory B cells. Nature Reviews Immunology. 15.3, 149-159 (2015).
5. Chen, K., Magri, G., Grasset, E. K. & Cerutti, A. Rethinking mucosal antibody responses:IgM, IgG and IgD join IgA. Nature Reviews Immunology. 20, 427-441 (2020).
6. Mesin, L. et al. Restricted clonality and limited germinal center reentry characterize memory B cell reactivation by boosting. Cell. 180.1, 92-106 (2020).
7. Chen, H. et al. BCR selection and affinity maturation in Peyer's patch germinal centres. Nature. 582, 421-425 (2020).
8. Ise, W. et al. T follicular helper cell-germinal center B cell interaction strength regulates entry into plasma cell or recycling germinal center cell fate. Immunity. 48.4, 702-715 (2018).
9. Komban, R.J. et al. Activated Peyer's patch B cells sample antigen directly from M cells in the subepithelial dome. Nat Commun. 10, 2423 (2019).
10. Song, S. & Matthias, P. D. The Transcriptional Regulation of Germinal Center Formation. Front Immunol. 10.3389 (2018).
11. Willis, SN. et al. Transcription factor IRF4 regulates germinal center cell formation through a B cell-intrinsic mechanism. J Immunol. 192(7), 3200-6 (2014).
12. Ahn, G. O. et al. Inhibition of Mac-1 (CD11b/CD18) enhances tumor response to radiation by reducing myeloid cell recruitment. Proc Natl Acad Sci. 107, 8363-8368 (2010)
13. Ding, C. et al. Integrin CD11b negatively regulates BCR signalling to maintain autoreactive B cell tolerance. Nat Commun. 4:2813 (2013).
14. Zhou, M. et al. Integrin CD11b Negatively Regulates B Cell Receptor Signaling to Shape Humoral Response during Immunization and Autoimmunity. The Journal of Immunology. 207.7, 1785-1797 (2021).
15. Kunisawa, J. et al. Microbe-dependent CD11b+ IgA+ plasma cells mediate robust early-phase intestinal IgA responses in mice. Nat Commun, 4, 1772 (2013)
16. Wei, M. et al. Mice carrying a knock-in mutation of Aicda resulting in a defect in somatic hypermutation have impaired gut homeostasis and compromised mucosal defense. Nature immunology. 12.3, 264-270 (2011).
17. Diamond, M. S. et al. ICAM-654 1 (CD54):a counter-receptor for Mac-1 (CD11b/CD18). The Journal of cell biology. 111(6), 3129-3139 (1990).
18. Figenschau, S. L. et al. ICAM1 expression is induced by proinflammatory cytokines and associated with TLS formation in aggressive breast cancer subtypes. Scientific Reports. 8, 11720 (2018).
19. Miyasaka, M., Tanaka, T. Lymphocyte trafficking across high endothelial venules:dogmas and enigmas. Nat Rev Immunol 4, 360-370 (2004).
20. Robinson, M. J. et al. The amount of BCL6 in B cells shortly after antigen engagement determines their representation in subsequent germinal centers. Cell reports. 30.5, 1530-1541 (2020).
21. Green, J. A. & Cyster, J. G. S1PR2 links germinal center confinement and growth regulation. Immunological reviews. 247.1, 36-51 (2012).
22. Shinkura, S. et al. Separate domains of AID are required for somatic hypermutation and class-switch recombination. Nature Immunology. 5, 707-712 (2004).
23. Pereira, J. P., Kelly, L. M., Xu, Y. & Cyster, J. G. EBI2 mediates B cell segregation between the outer and centre follicle. Nature. 460. 1122-1126 (2009).
24. Saito, M. et al. BCL6 suppression of BCL2 via Miz1 and its disruption in diffuse large B cell lymphoma. Proceedings of the National Academy of Sciences. 106(27), 11294-11299 (2009).
25. Ochiai, K. et al. Transcriptional regulation of germinal center B and plasma cell fates by dynamical control of IRF4. Immunity. 38.5, 918-929 (2013).
26. Nojima, T. et al. In-vitro derived germinal centre B cells differentially generate memory B or plasma cells in vivo. Nature Communications. 2, 465 (2011)
27. Lee, H. K., Lee, J. & Tobias, P. S. Two lipoproteins extracted from Escherichia coli K-12 LCD25 lipopolysaccharide are the major components responsible for Toll-like receptor 2-mediated signaling. The Journal of Immunology. 168(8), 4012-4017 (2002).
28. Lachmandas, T. et al. Microbial stimulation of different Toll-like receptor signalling pathways induces diverse metabolic programmes in human monocytes. Nat Microbiol. 2, 16246 (2017).
29. Borm, M., van Bodegraven, A., Mulder, C. et al. The effect of NOD2 activation on TLR2-mediated cytokine responses is dependent on activation dose and NOD2 genotype. Genes Immun. 9, 274-278 (2008).
30. Schwickert, T. A. et al. A dynamic T cell-limited checkpoint regulates affinity dependent B cell entry into the germinal center. J Exp Med. 208(6), 1243-1252 (2011).
31. Rammensee, H. G., Wiesmuller, K. H. & Loffler, M. W. A new synthetic toll-like receptor 1/2 ligand is an efficient adjuvant for peptide vaccination in a human volunteer. j. immunotherapy cancer. 7, 307 (2019).
32. List, C. et al. Toll-Like Receptor Ligand Based Adjuvant, PorB, Increases Antigen Deposition on Germinal Center Follicular Dendritic Cells While Enhancing the Follicular Dendritic Cells Network. Frontiers in Immunology. 11, 1254 (2020).
33. FARIDI, M. H. et al. CD11b activation suppresses TLR-dependent inflammation and autoimmunity in systemic lupus erythematosus. The Journal of clinical investigation, 127.4:1271-1283 (2017).
34. PASSOS, L. S. A. et al. Activation of human CD11b+ B1 B-cells by Trypanosoma cruzi-derived proteins is associated with protective immune response in human Chagas disease. Frontiers in immunology. 9, 3015 (2019)
35. Watanabe, T. et al. NOD2 is a negative regulator of Toll-like receptor 2-mediated T helper type 1 responses. Nat Immunol 5, 800-808 (2004)
36. Caruso, R. et al. NOD1 and NOD2:signaling, host defense, and inflammatory disease. Immunity 41.6, 898-908 (2014)
37. Takeda, K., Akira, S. Toll-like receptors in innate immunity. International immunology, 17(1), 1-14. (2005).
38. Yang, Z., Sullivan, B. M. & Allen, C. D. C. Fluorescent in vivo detection reveals that IgE+ B cells are restrained by an intrinsic cell fate predisposition. Immunity. 36(5), 857-872 (2012).
39. Nagai, T. et al. Expanded dynamic range of fluorescent indicators for Ca2+ by circularly permuted yellow fluorescent proteins. Proceedings of the National Academy of Sciences. 101(29), 10554-10559, (2004).
40. Yoshikawa, S. et al. Intravital imaging of Ca2+ signals in lymphocytes of Ca2+ biosensor transgenic mice:indication of autoimmune diseases before the pathological onset. Sci Rep. 6, 18738 (2016).
41. Miwa, Y., Tsubota, K. & Kurihara, T. Effect of midazolam, medetomidine, and butorphanol tartrate combination anesthetic on electroretinograms of mice. Molecular vision. 25, 645 (2019).
42. Jolly, C. J., Klix, N. & Neuberger, M. S. Rapid methods for the analysis of immunoglobulin gene hypermutation:application to transgenic and gene targeted mice. Nucleic acids research. 25.10,1913-1919 (1997).

## Claims

1. A composition for inducing migration of activated B cells to a germinal center, the composition comprising:
a substance that induces CD11b expression in B cells.

2. The composition according to claim 1, wherein the substance that induces the CD11b expression in B cells is a bacterium.

3. The composition according to claim 2, wherein the substance that induces the CD11b expression in B cells is a bacterium of the class γ Proteobacteria or class Bacteroidia.

4. The composition according to claim 3, wherein the substance that induces the CD11b expression in B cells is Escherichia coli, Salmonella enterica, Klebsiella pneumoniae, Bacteroides vulgatus, or a combination thereof.

5. The composition according to any one of claims 1 to 4, which is for treatment of a subject with a disease, disorder, or condition that is treated by activation of the migration of activated B cells to a germinal center.

6. The composition according to any one of claims 1 to 4, which is used for immunostimulation, maintenance or improvement of health, maintenance or improvement of mucosal flora, or treatment of an infectious disease.

7. The composition according to any one of claims 1 to 4, which is a mucosally administered vaccine adjuvant.

8. The composition according to any one of claims 1 to 6, which is in a form selected from the group consisting of a medicine, a food, a feed, a food additive, a feed additive, and a raw material composition thereof.

9. The composition according to claim 1, wherein the composition is administered together with an antigen.

10. The composition according to claim 9, wherein the composition is administered simultaneously with the antigen.

11. The composition according to claim 9, wherein the composition is administered separately from administration of the antigen.

12. The composition according to claim 1, which is a vaccine composition including an antigen.

13. The composition according to claims 9 to 12, wherein the antigen is a viral, bacterial, fungal, or parasitic antigen.

14. A method for inducing migration of activated B cells to a germinal center, the method comprising: preparing a composition including a substance that induces CD11b expression in B cells; and bringing the substance into contact with B cells, wherein the CD11b expression is induced in the B cells, and the contacts between the B cells and T cells and the B cells and dendritic cells are induced.
